# EUROPEAN PATENT APPLICATION

(11) **EP 4 682 271 A2**
(43) Date of publication of application: **21.01.2026**
(21) Application number: 25198136.1
(22) Date of filing: 12.03.2019
(51) Int. Cl.: C12R 1/91

(54) **USING INFECTIOUS NUCLEIC ACID TO TREAT CANCER**

(30) Priority: 12.03.2018 US 201862641814 P
(62) Divisional of application: 19767370.0
(71) Applicant: Mayo Foundation for Medical Education and Research, Rochester, MN 55905 (US)
(72) Inventor: SCHULZE, Autumn J., Rochester, MN 55901-0307 (US); RUSSELL, Stephen James, Rochester, MN 55902-6349 (US); ELSEDAWY, Noura B., Buffalo, NY 14209 (US)
(74) Representative: Fish & Richardson P.C.

(57) **Abstract**

This document provides methods and materials related to using infectious nucleic acid encoding viruses to reduce the number of viable cancer cells within a mammal. For example, methods for using infectious nucleic acid to treat cancer, engineered viral nucleic acid, and methods for making engineered viral nucleic acid are provided.

## Description

### CROSS-REFERENCE TO RELATED APPLICATIONS

This application claims the benefit of U.S. Patent Application Serial No. 62/641,814, filed on March 12, 2018. The disclosure of the prior application is considered part of (and is incorporated by reference in) the disclosure of this application.

### BACKGROUND

### 1. Technical Field

This document relates to methods and materials involved in treating cancer with viral nucleic acid (e.g., infectious nucleic acid encoding for a picornavirus).

### 2. Background Information

The genus enterovirus is a member of the family *Picornaviridae* and is comprised of four species of human enteroviruses, HEV-A to -D. Enteroviruses are small nonenveloped viruses with positive-sense single-stranded RNA genomes that are around 7500 nucleotides in length. Following cellular internalization, the virion is uncoated, and the genomic RNA is immediately translated into a single large polyprotein, which is processed by the virally-encoded protease (3C) to yield the capsid proteins and non-structural proteins involved in viral replication. A negative-sense strand RNA is synthesized from the positive-sense viral RNA by the virally encoded RNA-dependent RNA polymerase (RdRp-3D). The minus-strand RNA serves as a template for synthesizing more positive-sense RNA molecules that can be translated, replicated or packaged. The virus progeny accumulate in the cytoplasm until the virus-induces cell lysis releasing the progeny into the environment.

Coxsackievirus A21 (CVA21) is a naturally occurring HEV-C picornavirus known to cause upper respiratory infections or in more severe cases myositis in humans. Infants and immunocompromised individuals have a greater chance to develop more severe complications. Intracellular adhesion molecule 1 (ICAM-1) is the main receptor for the virus, and CVA21 has shown potent oncolytic activity against a variety of ICAM-1 expressing cancer cells. However, immunocompromised mice bearing human melanoma and myeloma xenografts develop rapid onset lethal myositis (presented as flaccid limb paralysis) following treatment with CVA21 formulated as virus particles or as infectious nucleic acid.

### SUMMARY

This document provides methods and materials related to using nucleic acid (e.g., infectious nucleic acid) encoding viruses to reduce the number of viable cancer cells within a mammal. For example, this document provides methods for using infectious nucleic acid to treat cancer, engineered viral nucleic acid, and methods for making engineered viral nucleic acid.

MicroRNA-targeting can be used to regulate the tropism of positive-sense RNA viruses. This technique exploits tissue-specific microRNAs (miRNA) expressed in host cells where viral replication is undesirable (Kelly et al., Nat. Med., 14:1278-1283 (2008); Barnes et al., Cell Host Microbe, 4:239-248 (2008); and Ruiz et al., J. Virol., 90:4078-4092 (2016)). MiRNA target sequences can be inserted into the viral genome such that the genome and mRNAs are recognized by the host miRNAs and subsequently degraded or translationally repressed, preventing viral replication and toxicity. Cancer cells often have dysregulated miRNA levels, allowing permissive virus replication and subsequent tumor cell destruction.

The shapes of RNA molecules, including viral genomes, are specifically designed to regulate stability, intra- and inter-molecular interactions, and activity. Therefore, it is reasonable to speculate that insertion of additional nucleotides (e.g., miRNA targets) anywhere within the viral genome has the potential to significantly offend a variety of the biological properties of the virus. For example, microRNA target sequences can be inserted into the viral genome in the 3' UTR, and the rescued virus can replicate with kinetics similar to the unmodified virus, maintain oncolytic activity against human xenografts *in vivo,* and reduce toxicity in normal tissues as described elsewhere (Kelly et al., Nat. Med., 14:1278-1283 (2008)). However, rescue of this virus following transfection of RNA transcripts encoding this modified viral genome was significantly delayed.

In addition, wild-type viruses can exhibit significant toxicity. For example, injection of RNA transcripts encoding the wild-type viral genome into mice bearing human melanoma or myeloma xenografts nucleated a spreading viral infection resulting in tumor reduction and lethal toxicity as described elsewhere (Hadac et al., Molecular Therapy, 19(6):1041-1047 (2011)). In contrast, injection of RNA transcripts encoding the Kelly *et al.* miRNA-targeted viral genome did not mount a spreading infection and thus does not have therapeutic value as infectious nucleic acid.

As described herein, microRNA-targeted oncolytic viruses were formulated as infectious nucleic acid using a coxsackievirus A21 backbone. Various alternative insertion mechanisms were designed, and the viral genomes were constructed. In addition, the virus rescue kinetics from RNA transcripts, the rescued virus replication kinetics, microRNA-target stability, oncolytic activity, and toxicity were evaluated; each in comparison to the unmodified genome and the Kelly *et al.* miRNA-targeted genome. Each tested construct exhibited enhanced viral rescue kinetics compared to the Kelly *et al.* construct and maintained oncolytic activity. In addition, one tested construct (designated CVA21-ΔV₂ₓ herein) ameliorated toxicity and significantly prolonged overall survival.

The CVA21-ΔV₂ₓ construct is an example of a microRNA-targeted viral genome formulated as infectious nucleic acid that exhibits an acceptable therapeutic index that was experimentally validated. The constructs provided herein can be used for anti-cancer therapy, vaccination with enhanced safety, or segregation of viral growth in various producer and target cells, facilitating manufacturing and experimental evaluation of the virus life cycle and the role of different cells in pathogenesis. Additionally, the techniques for microRNA-targeting provided herein can be used with other type I IRES encoding picornaviruses.

In general, one aspect of this document features a nucleic acid construct comprising (or consisting essentially of or consisting of) an infectious nucleic acid comprising (or consisting essentially of or consisting of) a picornavirus genome comprising (or consisting essentially of or consisting of) one or more heterologous sequence elements of 20 or more bases, where the specific infectivity of the construct is sufficient to initiate a spreading picornavirus infection when administered to a living mammal, where the specific infectivity of the construct is of similar magnitude to the specific infectivity of a comparable construct lacking the one or more heterologous sequence elements. The mammal can be a human. The construct can be formulated as plasmid DNA. The construct can be formulated as an RNA molecule. At least one of the one or more heterologous sequence elements can be a microRNA response element. A microRNA target element of the microRNA response element can comprise (or can consist essentially of or can consist of) at least a region of complementarity to a microRNA present in non-cancer cells. At least one of the one or more heterologous sequence elements can be a tissue-specific microRNA response element. At least one of the one or more heterologous sequence elements can be a muscle-specific, brain-specific, or heart-specific microRNA response element. At least one of the one or more heterologous sequence elements can be inserted into the 5' non-coding region of the picornavirus genome as a substitution for nucleotides within a scanning region. The picornavirus genome can comprise a type I internal ribosome entry site. The picornavirus genome can be a coxsackievirus, poliovirus, echovirus, rhinovirus, or enterovirus genome. The picornavirus genome can be a coxsackievirus A21 genome. The picornavirus genome can comprise a microRNA target element for miR-133. The picornavirus genome can comprise more than one microRNA target element for miR-133. The picornavirus genome can comprise a microRNA target element for miR-206. The picornavirus genome can comprise more than one microRNA target element for miR-206. The picornavirus genome can comprise more than one microRNA target element for miR-133 and more than one microRNA target element for miR-206. The picornavirus genome can be a coxsackievirus A21 genome, and the picornavirus genome can lack at least 20 nucleotides from position 631 to position 698 of a wild-type coxsackievirus A21 genome. The picornavirus genome can be a coxsackievirus A21 genome, and the picornavirus genome can lack nucleotides 631 to 698 of a wild-type coxsackievirus A21 genome. In some cases, the nucleic acid construct can be DNA, and the nucleic acid construct can encode a ribozyme. The ribozyme can be designed to cleave a 5' portion of RNA from an RNA molecule transcribed from the nucleic acid construct, thereby creating a ribozyme-cleaved RNA. The ribozyme-cleaved RNA can have a 5' end with no ribonucleotides that are not present in a picornavirus genome. In some cases, the nucleic acid construct can be RNA, and the nucleic acid construct can include a ribozyme. The ribozyme can be designed to cleave a 5' portion of RNA from the nucleic acid construct, thereby creating a ribozyme-cleaved RNA. The ribozyme-cleaved RNA can have a 5' end with no ribonucleotides that are not present in a picornavirus genome. In some cases, the nucleic acid construct can be DNA, and the nucleic acid construct can include a restriction endonuclease cut site. The restriction endonuclease cut site can be designed to allow for cleavage, via a restriction endonuclease, of a 3' portion of said nucleic acid construct, thereby creating a restriction endonuclease-cleaved nucleic acid construct. The restriction endonuclease-cleaved nucleic acid construct can encode a virus having a 3' end with less than 10 ribonucleotides that are not present in a picornavirus genome (e.g., a picornavirus genome having a 3' end with no ribonucleotides that are not present in a picornavirus genome). In some cases, the nucleic acid construct can be DNA, the nucleic acid construct can encode a ribozyme, and the nucleic acid construct can include a restriction endonuclease cut site. The ribozyme can be designed to cleave a 5' portion of RNA from an RNA molecule transcribed from the nucleic acid construct, thereby creating a ribozyme-cleaved RNA. The ribozyme-cleaved RNA can have a 5' end with no ribonucleotides that are not present in a picornavirus genome. The restriction endonuclease cut site can be designed to allow for cleavage, via a restriction endonuclease, of a 3' portion of the nucleic acid construct, thereby creating a restriction endonuclease-cleaved nucleic acid construct. The restriction endonuclease-cleaved nucleic acid construct can encode a virus having a 3' end with less than 10 ribonucleotides that are not present in a picornavirus genome (e.g., a virus having a 3' end with no ribonucleotides that are not present in a picornavirus genome).

In another aspect, this document features a method of reducing the number of cancer cells within a living mammal. The method comprises (or consists essentially of or consists of) administering a construct to the mammal. The mammal can be a human. The construct can be formulated as plasmid DNA. The construct can be formulated as an RNA molecule. At least one of the one or more heterologous sequence elements can be a microRNA response element. A microRNA target element of the microRNA response element can comprise (or can consist essentially of or can consist of) at least a region of complementarity to a microRNA present in non-cancer cells. At least one of the one or more heterologous sequence elements can be a tissue-specific microRNA response element. At least one of the one or more heterologous sequence elements can be a muscle-specific, brain-specific, or heart-specific microRNA response element. At least one of the one or more heterologous sequence elements can be inserted into the 5' non-coding region of the picornavirus genome as a substitution for nucleotides within a scanning region. The picornavirus genome can comprise a type I internal ribosome entry site. The picornavirus genome can be a coxsackievirus, poliovirus, echovirus, rhinovirus, or enterovirus genome. The picornavirus genome can be a coxsackievirus A21 genome. The picornavirus genome can comprise a microRNA target element for miR-133. The picornavirus genome can comprise more than one microRNA target element for miR-133. The picornavirus genome can comprise a microRNA target element for miR-206. The picornavirus genome can comprise more than one microRNA target element for miR-206. The picornavirus genome can comprise more than one microRNA target element for miR-133 and more than one microRNA target element for miR-206. The picornavirus genome can be a coxsackievirus A21 genome, and the picornavirus genome can lack at least 20 nucleotides from position 631 to position 698 of a wild-type coxsackievirus A21 genome. The picornavirus genome can be a coxsackievirus A21 genome, and the picornavirus genome can lack nucleotides 631 to 698 of a wild-type coxsackievirus A21 genome. The cancer cells can be melanoma, myeloma, pancreatic, prostate, bladder, non-small cell lung, or breast cancer cells. The administering step can result in a reduced number of non-cancerous cells present within the mammal undergoing cell lysis following the administering step as compared to the number of non-cancerous cells that undergo lysis when the comparable construct is administered to a comparable mammal. The administering step can result in a similar number or an increased number of cancerous cells present within the living mammal undergoing cell lysis following the administering step as compared to the number of cancerous cells that undergo lysis when the comparable construct is administered to a comparable mammal.

In another aspect, this document features a method of reducing the number of cancer cells within a living mammal. The method comprises (or consists essentially of or consists of) administering a construct to the mammal, where the cancer cells undergo lysis as a result of unencumbered synthesis of corresponding picornaviruses from the construct, thereby reducing the number of cancer cells within the living mammal. The mammal can be a human. The construct can be formulated as plasmid DNA. The construct can be formulated as an RNA molecule. At least one of the one or more heterologous sequence elements can be a microRNA response element. A microRNA target element of the microRNA response element can comprise (or can consist essentially of or can consist of) at least a region of complementarity to a microRNA present in non-cancer cells. At least one of the one or more heterologous sequence elements can be a tissue-specific microRNA response element. At least one of the one or more heterologous sequence elements can be a muscle-specific, brain-specific, or heart-specific microRNA response element. At least one of the one or more heterologous sequence elements can be inserted into the 5' non-coding region of the picornavirus genome as a substitution for nucleotides within a scanning region. The picornavirus genome can comprise a type I internal ribosome entry site. The picornavirus genome can be a coxsackievirus A21, poliovirus, echovirus, rhinovirus, or enterovirus genome. The picornavirus genome can be a coxsackievirus A21 genome. The picornavirus genome can comprise a microRNA target element for miR-133. The picornavirus genome can comprise more than one microRNA target element for miR-133. The picornavirus genome can comprise a microRNA target element for miR-206. The picornavirus genome can comprise more than one microRNA target element for miR-206. The picornavirus genome can comprise more than one microRNA target element for miR-133 and more than one microRNA target element for miR-206. The picornavirus genome can be a coxsackievirus A21 genome, and the picornavirus genome can lack at least 20 nucleotides from position 631 to position 698 of a wild-type coxsackievirus A21 genome. The picornavirus genome can be a coxsackievirus A21 genome, and the picornavirus genome can lack nucleotides 631 to 698 of a wild-type coxsackievirus A21 genome. The cancer cells can be melanoma, myeloma, or breast cancer cells. The administering step can result in a reduced number of non-cancerous cells present within the mammal undergoing cell lysis following the administering step as compared to the number of non-cancerous cells that undergo lysis when the comparable construct is administered to a comparable mammal. The administering step can result in a similar number or an increased number of cancerous cells present within the living mammal undergoing cell lysis following the administering step as compared to the number of cancerous cells that undergo lysis when the comparable construct is administered to a comparable mammal.

In another aspect, this document features an isolated infectious nucleic acid encoding a coxsackievirus, where the infectious nucleic acid lacks at least 20 nucleotides from position 631 to position 698 of a wild-type coxsackievirus A21 genome (e.g., the Kuykendall CVA21 strain), and where the infectious nucleic acid comprises a microRNA target element for a muscle-specific microRNA that is located between a VI domain and an authentic translation start site of the infectious nucleic acid. The infectious nucleic acid can lack the nucleotides from position 631 to position 698. The muscle-specific microRNA can be miR-133 or miR-206. The infectious nucleic acid can comprise the microRNA target element between a first position and a second position, where the first position corresponds to position 631 of the wild-type coxsackievirus A21 genome, and where the second position corresponds to position 699 of the wild-type coxsackievirus A21 genome. In some cases, the infectious nucleic acid can be DNA, and the infectious nucleic acid can include a restriction endonuclease cut site. The restriction endonuclease cut site can be designed to allow for cleavage, via a restriction endonuclease, of a 3' portion of the infectious nucleic acid, thereby creating a restriction endonuclease-cleaved infectious nucleic acid. The restriction endonuclease-cleaved infectious nucleic acid can encode a coxsackievirus having a 3' end with less than 10 ribonucleotides that are not present in a picornavirus genome (e.g., a coxsackievirus having a 3' end with no ribonucleotides that are not present in a picornavirus genome). In some cases, the infectious nucleic acid can be RNA, the infectious nucleic acid can encode a ribozyme, and the infectious nucleic acid can include a restriction endonuclease cut site. The ribozyme can be designed to cleave a 5' portion of RNA from an RNA molecule transcribed from the infectious nucleic acid, thereby creating a ribozyme-cleaved RNA. The ribozyme-cleaved RNA can have a 5' end with no ribonucleotides that are not present in a picornavirus genome. The restriction endonuclease cut site can be designed to allow for cleavage, via a restriction endonuclease, of a 3' portion of RNA from the infectious nucleic acid, thereby creating a restriction endonuclease-cleaved infectious nucleic acid. The restriction endonuclease-cleaved infectious nucleic acid can encode a coxsackievirus having a 3' end with less than 10 ribonucleotides that are not present in a picornavirus genome (e.g., a coxsackievirus comprising a 3' end with no ribonucleotides that are not present in a picornavirus genome).

In another aspect, this document features a method for treating cancer present in a mammal. The method comprises (or consists essentially of or consists of) administering, to the mammal, an effective amount of infectious nucleic acid encoding a coxsackievirus, where the infectious nucleic acid lacks at least 20 nucleotides from position 631 to position 698 of a wild-type coxsackievirus A21 genome, and where the infectious nucleic acid comprises a microRNA target element for a muscle-specific microRNA that is located between a VI domain and an authentic translation start site of the infectious nucleic acid. The mammal can be a human. The cancer can be melanoma, myeloma, pancreatic, prostate, bladder, non-small cell lung, or breast cancer. The infectious nucleic acid can lack the nucleotides from position 631 to position 698. The muscle-specific microRNA can be miR-133 or miR-206. The infectious nucleic acid can comprise the microRNA target element between a first position and a second position, where the first position corresponds to position 631 of the wild-type coxsackievirus A21 genome, and where the second position corresponds to position 699 of the wild-type coxsackievirus A21 genome.

In another aspect, this document features a method for making infectious RNA comprising a picornavirus genome comprising one or more heterologous sequence elements of 20 or more bases, where the specific infectivity of the infectious RNA is sufficient to initiate a spreading picornavirus infection when administered to a living mammal, where the specific infectivity of the infectious RNA is of similar magnitude to the specific infectivity of a comparable infectious RNA lacking the one or more heterologous sequence elements. The method comprises (or consists essentially of or consists of) providing an DNA construct encoding the infectious RNA, where the DNA construct encodes a ribozyme, and where the nucleic acid construct includes a restriction endonuclease cut site; and contacting the DNA construct with a restriction endonuclease under conditions where at least a portion of the DNA construct is removed, thereby producing a restriction endonuclease-cleaved DNA construct; and where the restriction endonuclease-cleaved DNA construct encodes an infectious RNA having non-picornavirus RNA located at a 5' end, and where the ribozyme cleaves the non-picornavirus RNA located at the 5' end to generate the infectious RNA. The infectious RNA encoded by the restriction endonuclease-cleaved DNA construct can include a 3' end with less than 10 ribonucleotides that are not present in a picornavirus genome. The infectious RNA encoded by the restriction endonuclease-cleaved DNA construct can include a 3' end with no ribonucleotides that are not present in a picornavirus genome. The infectious RNA can include a 5' end with no ribonucleotides that are not present in a picornavirus genome.

In another aspect, this document features an immunocompetent model that can be infected by infectious nucleic acid encoding a virus, where the immunocompetent model includes a mouse cell expressing a human ICAM-1. The mouse cell can be a murine melanoma B16-F10 cell.

The term "specific infectivity" as used herein refers to the ratio between infectious viruses to total nucleic acid molecules (i.e., the number of plaque forming units obtained from a set copy number of viral genomes).

The term "scanning region" as used herein refers to the space between silent or cryptic AUG sites in internal ribosome entry sites and the authentic AUG. Ribosomes scan this region prior to translation initiation. This space generally ranges from 35-160 nucleotides in picornaviruses.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

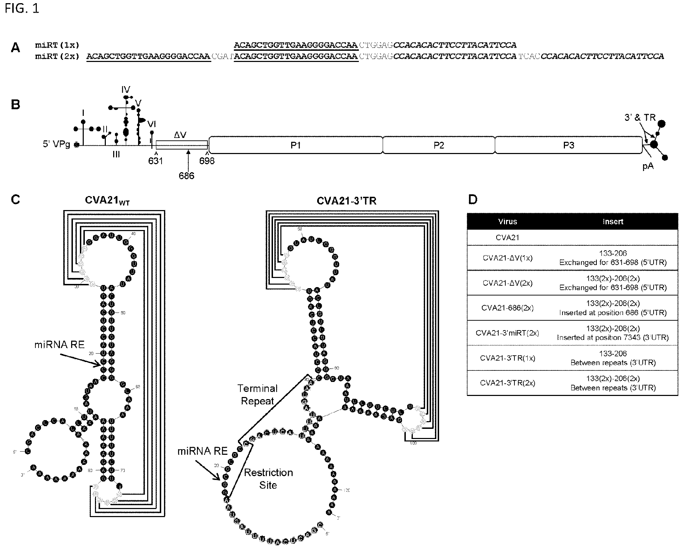

Figure 1. Schematic representation of potential UTR-localized insertion sites for microRNA response element (RE). (A) Sequences of muscle-detargeting response elements. Top = SEQ ID NO:1; bottom = SEQ ID NO:2. 1X constructs contain one copy each, and 2X constructs contain two copies each of target sequences complementary to miR-133 (underlined) and miR-206 (italics). (B) Diagram of CVA21 genome with RE insertion sites analyzed. Domains I to VI in the 5' UTR and response element insertion sites are labeled. The cryptic AUG site in domain VI is represented by a double line. (C) Left: RNA secondary structure model for CVA21_{WT} 3' UTR and location for RE constructed by Kelly *et al.* (SEQ ID NO:3). Right: Secondary structure model for CVA21-3'TR constructs (SEQ ID NO:4). Residues involved in forming the "kissing domain" are shown with interconnecting lines. Residues replicated to form the TR are labeled. (D) Nomenclature for all microRNA-targeted CVA21 constructs analyzed.

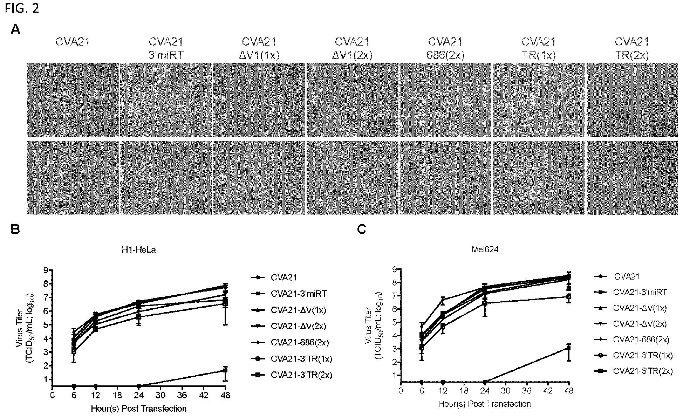

Figure 2. Transfection of miRT-CVA21 infectious RNA produces virus progeny at rates similar to wild-type CVA21 RNA. (A) Upper panel: H1-HeLa cells 72 hours post transfection with 2.5 µg of infectious RNA encoding CVA21 or miRT-CVA21. Lower panel: H1-HeLa cells 24 hours post infection with cleared lysates collected from transfected cells. (B-C) Time-course production of infectious virus in H1-HeLa cells (B) and Mel624 (C) cells transfected with 1 µg of infectious RNA encoding CVA21 or miRT-CVA21. Experiments were repeated at least in triplicate. Data is represented as mean viral titer +/- standard deviation.

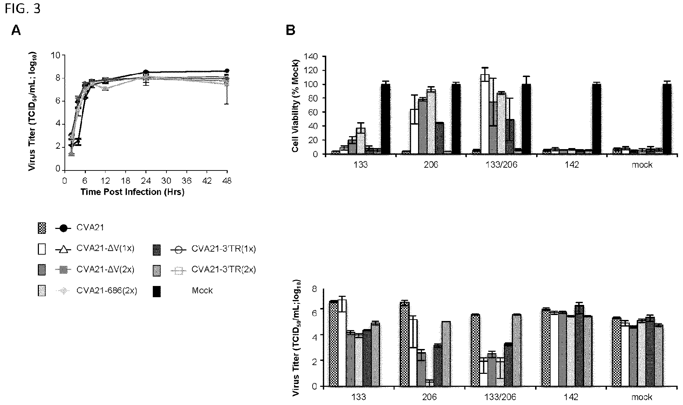

Figure 3. Localization of RE within the 5' UTR enhances regulation of viral tropism. (A) One-step growth curve of unmodified and new miRT-CVA21. (B) Upper: Viability of cells 24 hours post infection at an MOI of 1 with unmodified or new miRT-CVA21 of H1-HeLa cells pretreated with 100 nM miRNA mimics (labeled on x-axis). Lower: Viral titers of supernatants collected from the corresponding infections. Experiments were conducted in triplicate and data is represented as mean viability or mean virus titer +/- standard deviation.

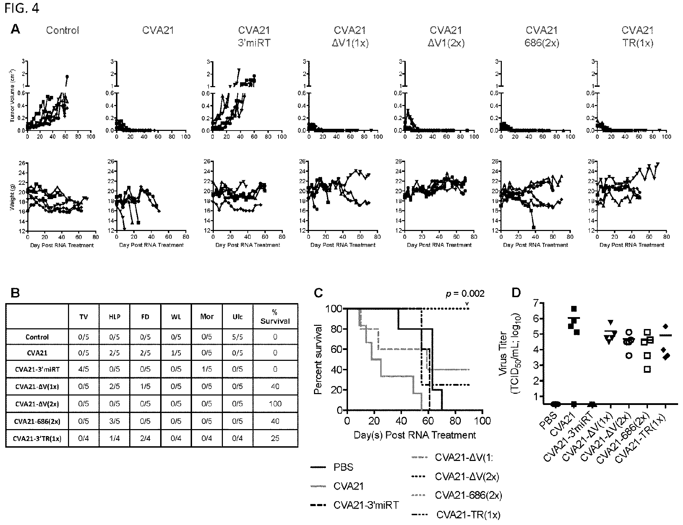

Figure 4. Treatment with CVA21-ΔV(2x) RNA results in complete tumor regression without causing toxicity. (A) Tumor volume and weight measurements from SCID mice bearing subcutaneous Mel624 tumors following IT treatment with saline (*n =* 5) or 30 µg of RNA transcripts encoding CVA21 (*n =* 5), CVA21-3'miRT (*n* = 5), CVA21-ΔV(1x) (*n =* 5), CVA21-ΔV(2x) (*n =* 5), CVA21-686(2x) (*n =* 5), or CVA21-3'TR(1x) (*n* = 4). (B) Proportion of mice that develop toxicities in each group and overall percent survival. (C) Kaplan-Meier survival graphs of treated mice. Statistical significance of survival between saline and CVA21-ΔV(2x) RNA treated mice was compared using a log-rank test. (D) Viral loads in sera collected on day 7 post therapy from treated mice. Horizontal lines represent mean viral titers.

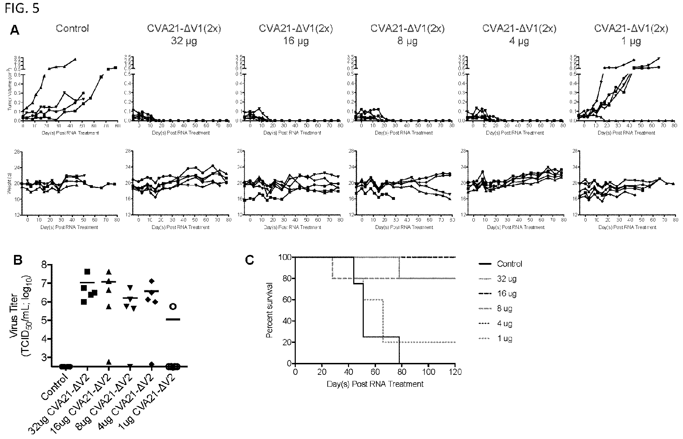

Figure 5. Oncolytic activity of CVA21-ΔV(2x) RNA is dose dependent. (A) Tumor volume and weight measurements from SCID mice bearing subcutaneous Mel624 tumors following a single IT injection of saline (*n* = 4), 1 µg (*n* = 5), 4 µg (*n* = 5), 8 µg (*n* = 5), 16 µg (*n* = 5), or 32 µg (*n* = 5) of CVA21-ΔV(2x) RNA. (B) Viral loads in sera collected on day 9 post RNA administration from mice treated in A. (C) Kaplan-Meier survival graphs of treated mice.

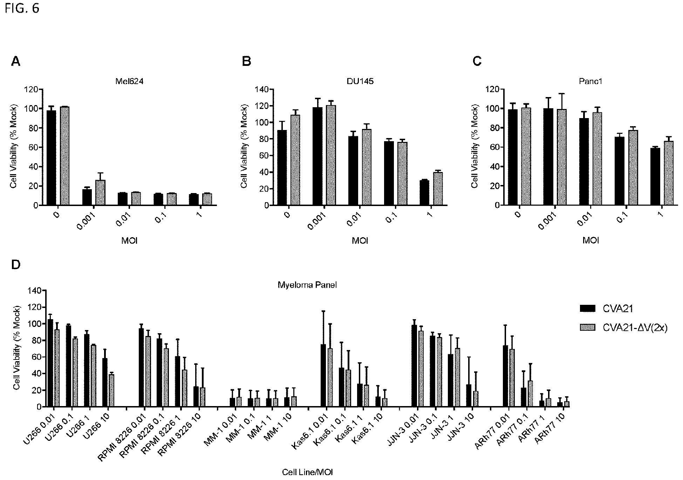

Figure 6. Cytotoxicity of CVA21 and CVA21-ΔV(2x) in a panel of tumor cell lines. 1 x 10⁴ cells per well of Mel624 human melanoma (A), DU145 human prostate (B), Panc1 human pancreatic ductal adenocarcinoma (C), U266, RPMI-8226, MM-1, Kas6.1, JJN-3, or ARh77 human myeloma (D) cells were plated in 96-well tissue culture dishes. The cells were infected with CVA21 or CVA21-ΔV(2x) at an MOI of 0.001, 0.01, 0.1, 1, or 10 for 2 hours at 37 °C in serum-free media. Following infection, the media and unincorporated virus was removed and replaced with 100 µL complete growth media, and the cells were incubated at 37 °C. 72 hours post infection, the cells were assayed for proliferation using a 3-(4,5-dimethylthiazolyl-2)-2,5-Diphenyltetrazolium bromide (MTT) kit (ATCC, Manassas, VA). Myeloma panel (*n =* 5). All other lines (*n =* 3). Data is represented as mean percent cell viability normalized to mock infected cells +/- standard deviation.

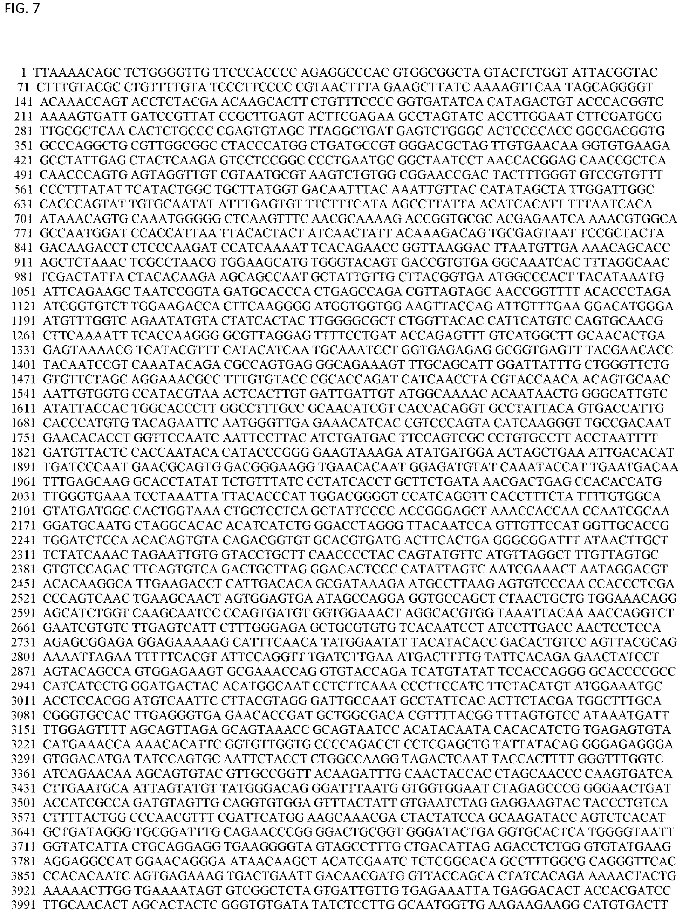

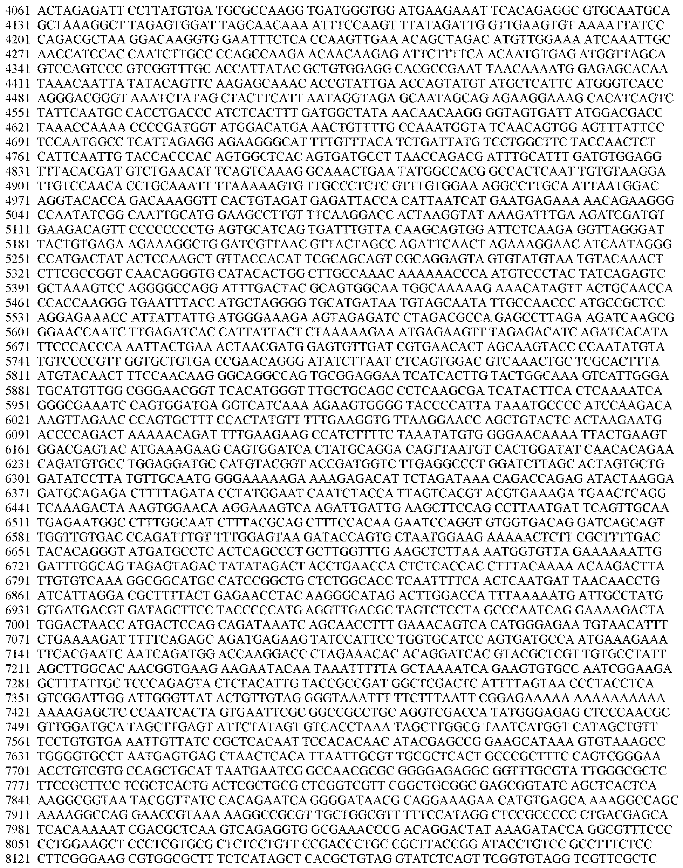

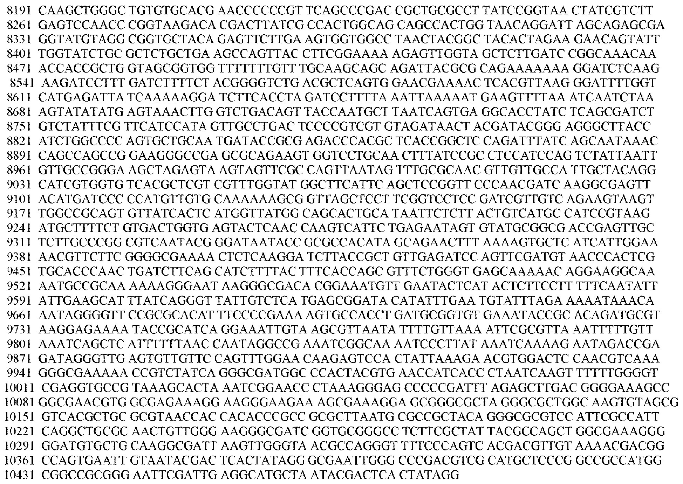

Figure 7 is a sequence listing of the DNA encoding infectious nucleic acid of wild type CVA21 and the encoded RNA (SEQ ID NO:5).

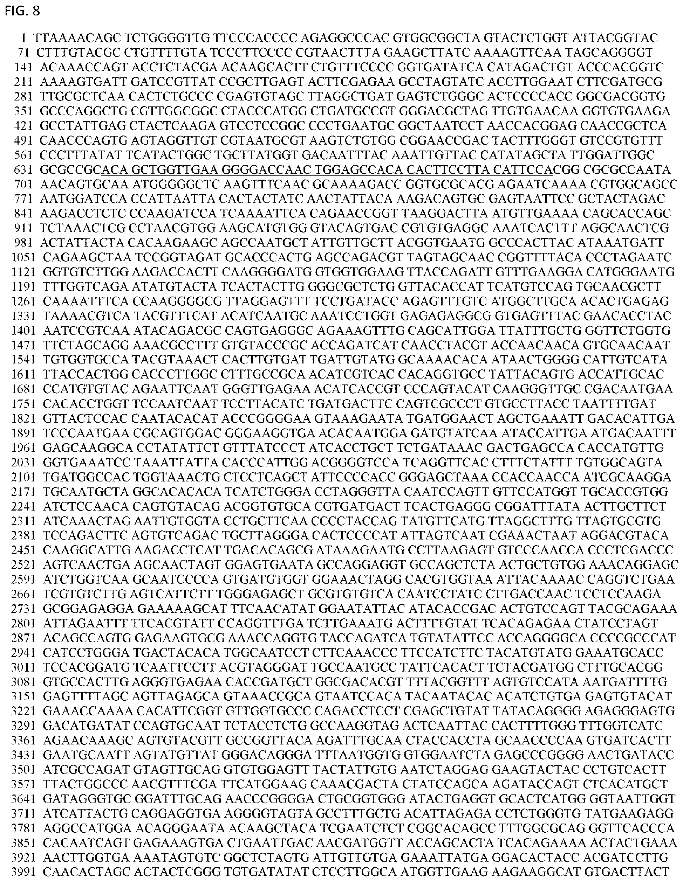

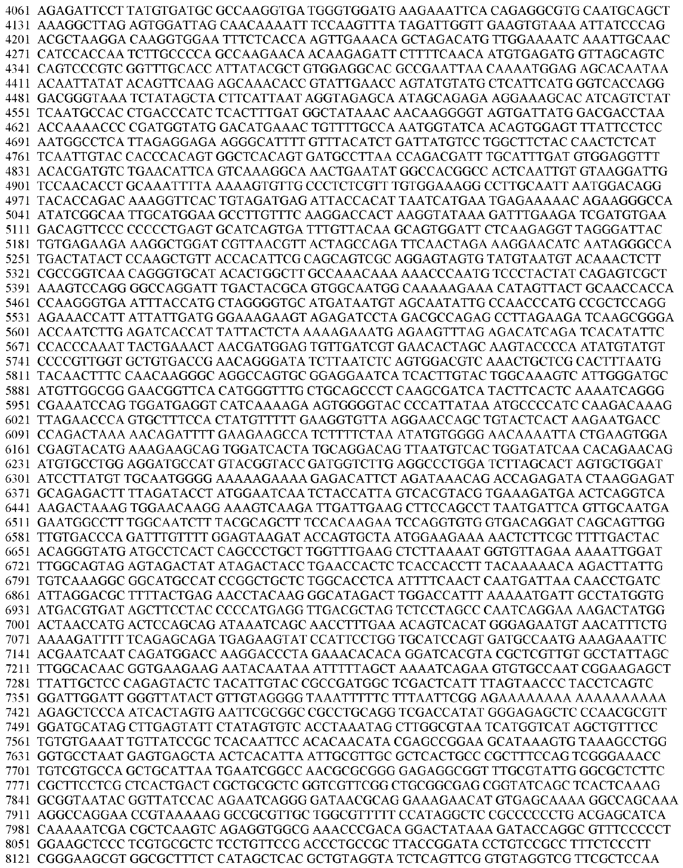

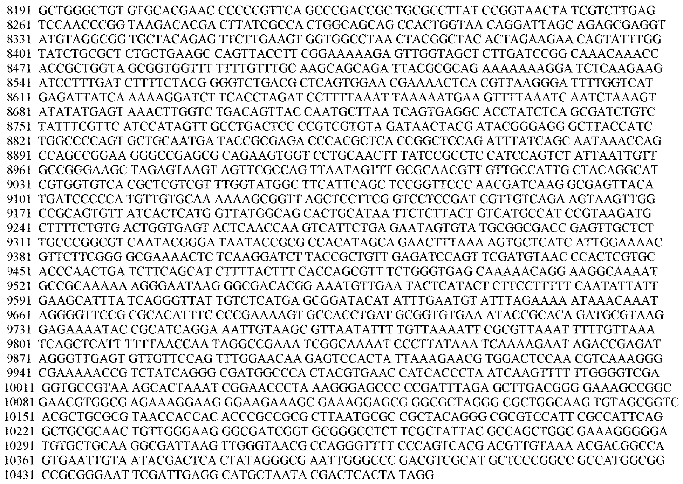

Figure 8 is a sequence listing of the DNA encoding infectious nucleic acid of CVA21-ΔV(1x) and the encoded RNA (SEQ ID NO:6). miRT(1x) is underlined.

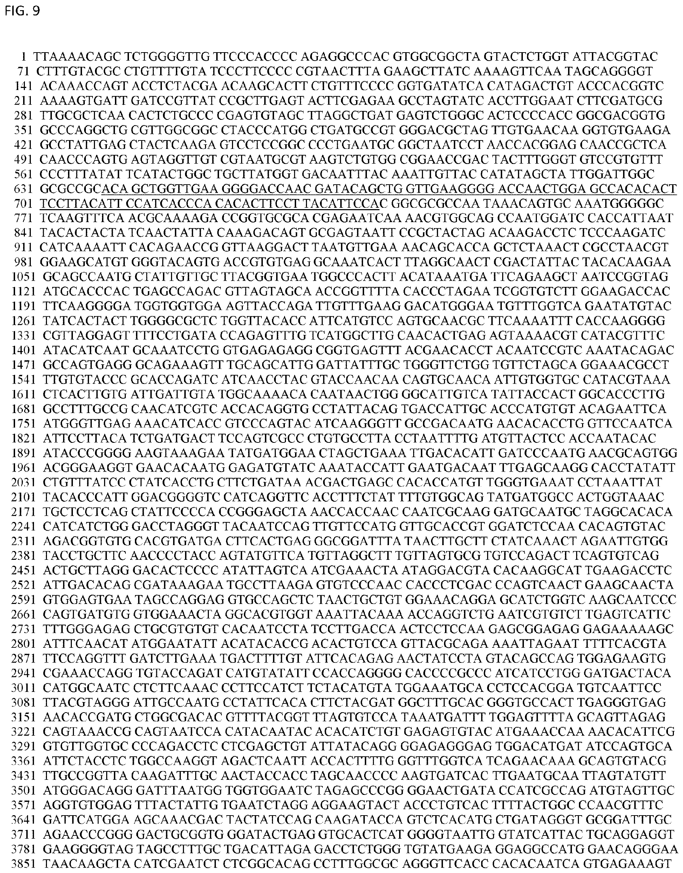

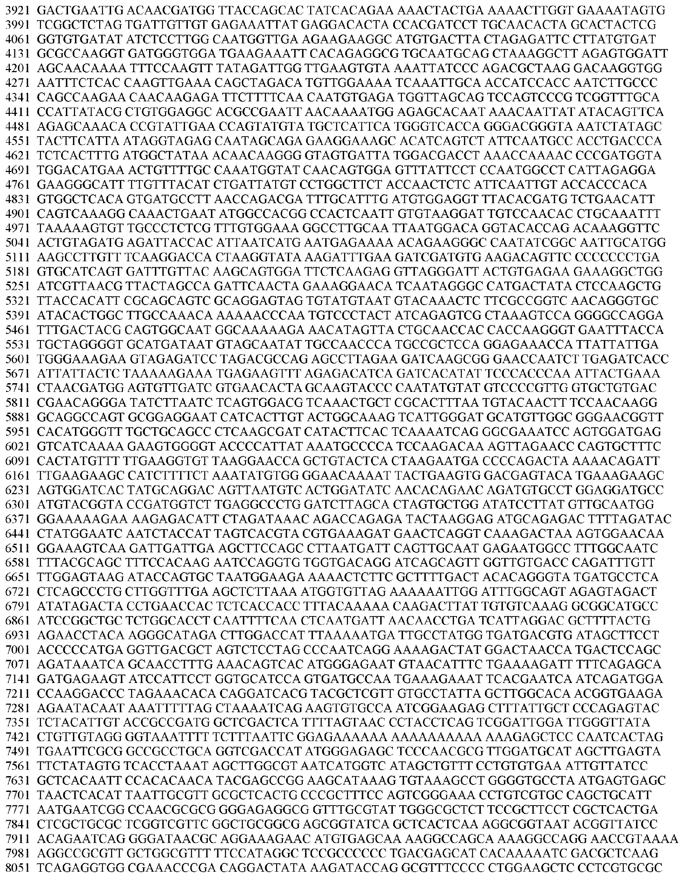

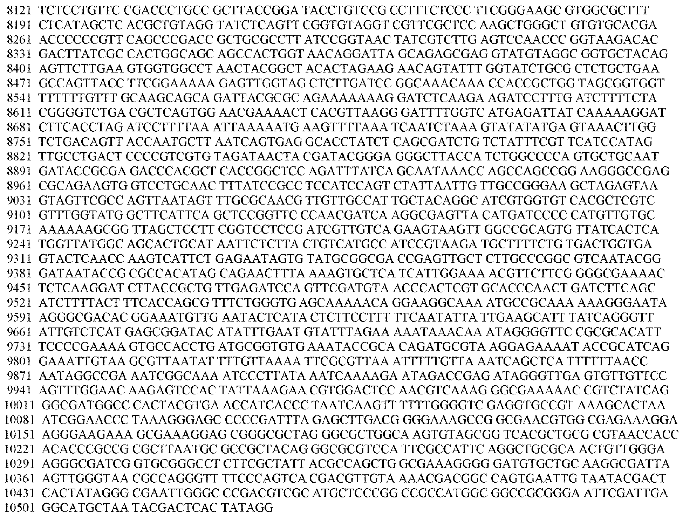

Figure 9 is a sequence listing of the DNA encoding infectious nucleic acid of CVA21-ΔV(2x) and the encoded RNA (SEQ ID NO:7). miRT(2x) is underlined.

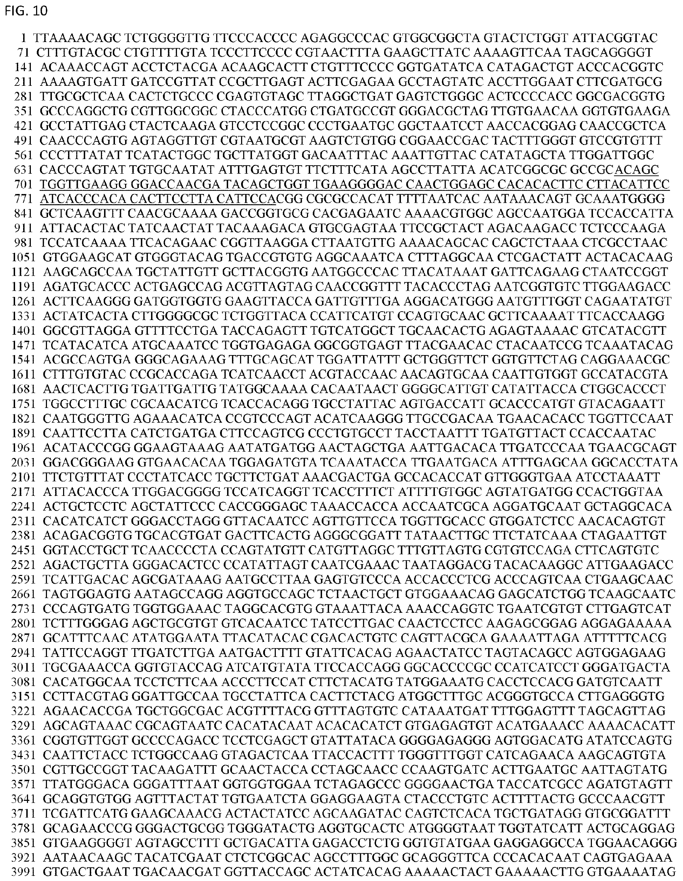

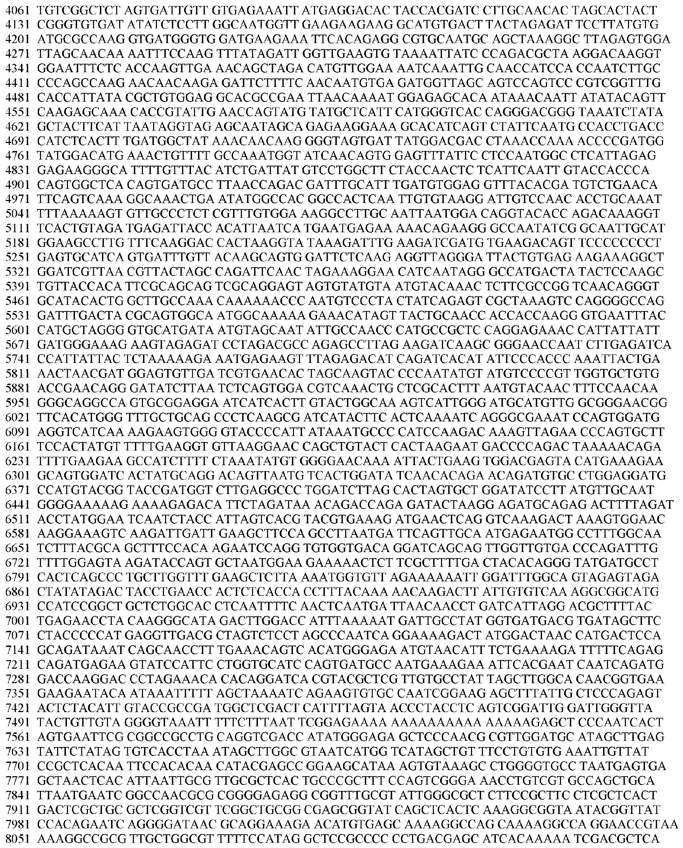

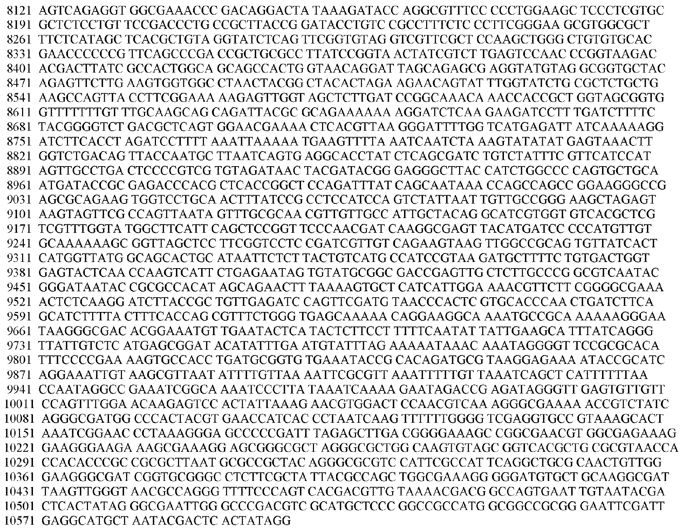

Figure 10 is a sequence listing of the DNA encoding infectious nucleic acid of CVA21-686(2x) and the encoded RNA (SEQ ID NO:8). miRT(2x) is underlined.

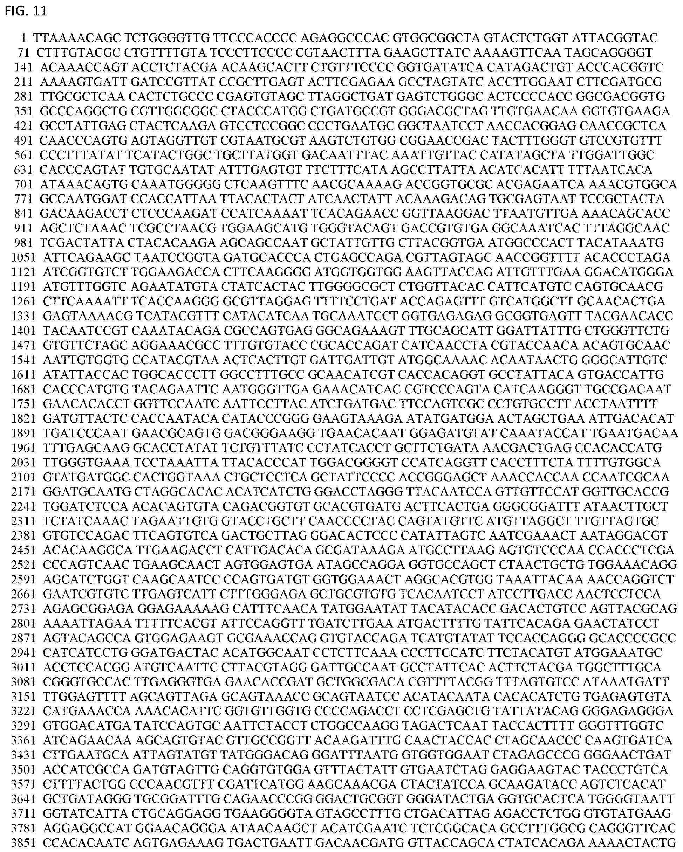

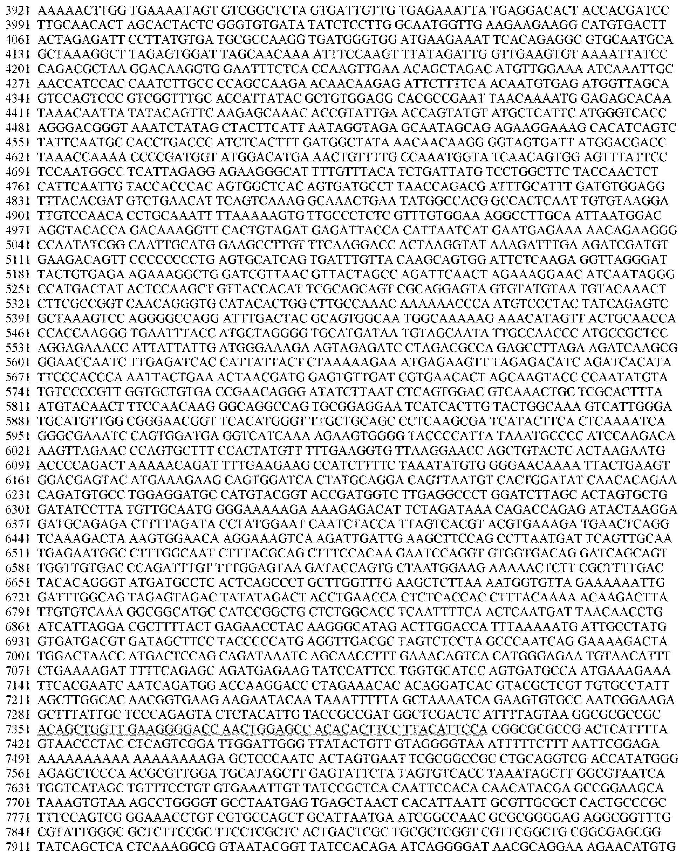

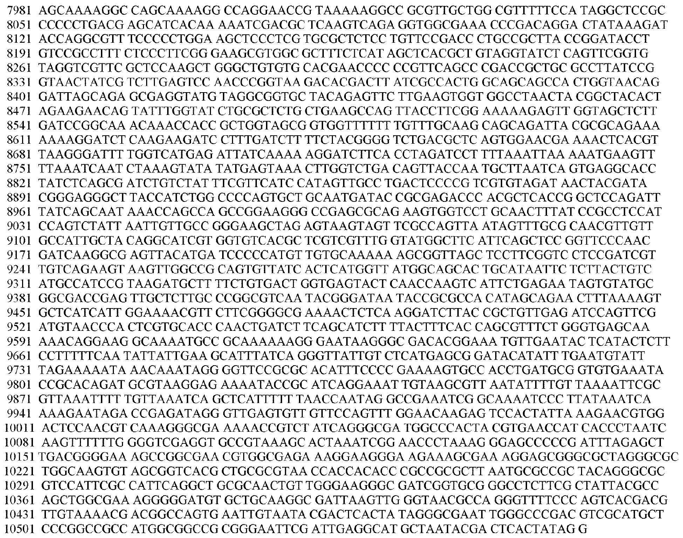

Figure 11 is a sequence listing of the DNA encoding infectious nucleic acid of CVA21-3'TR(1x) and the encoded RNA (SEQ ID NO:9). miRT(1x) is underlined.

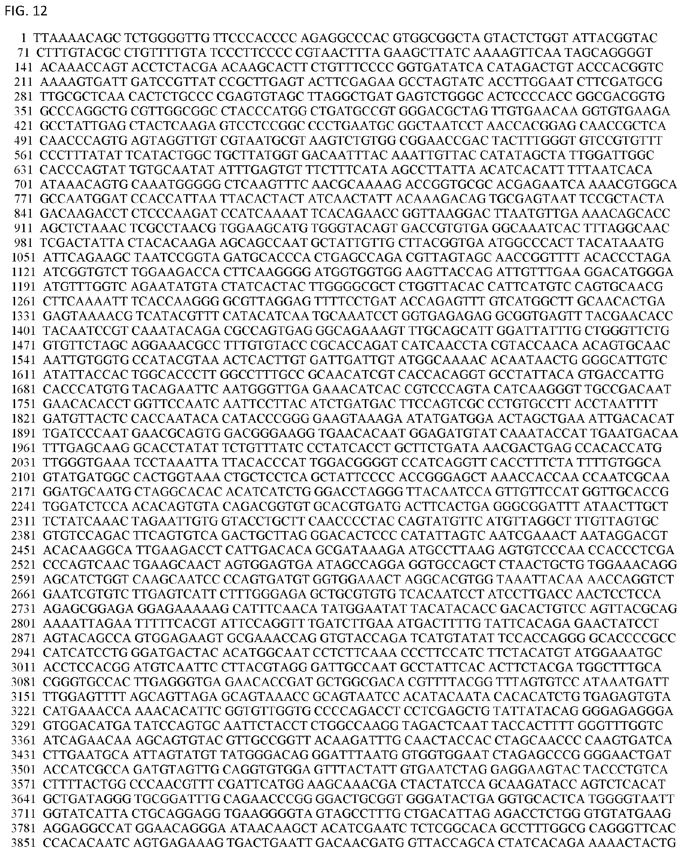

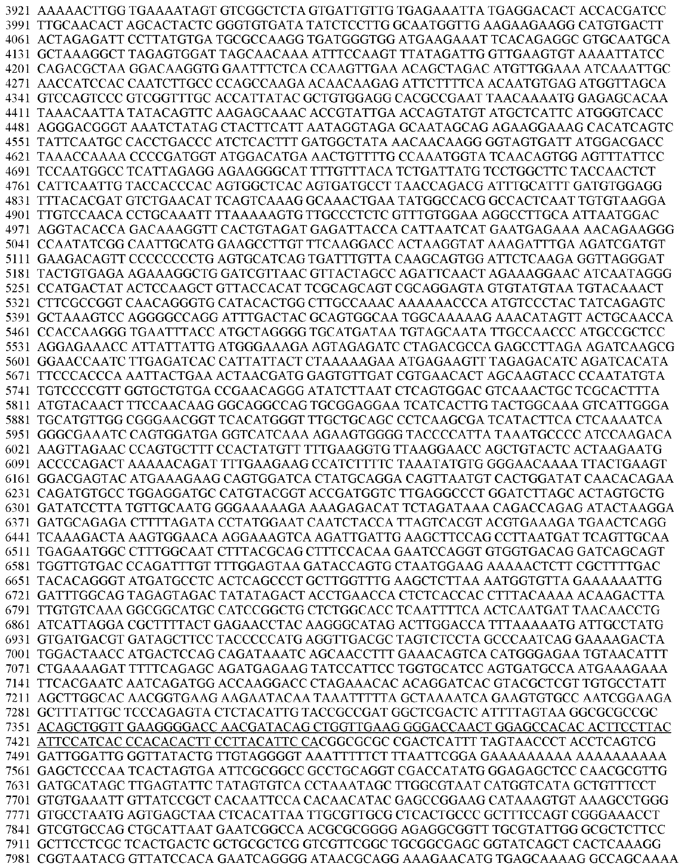

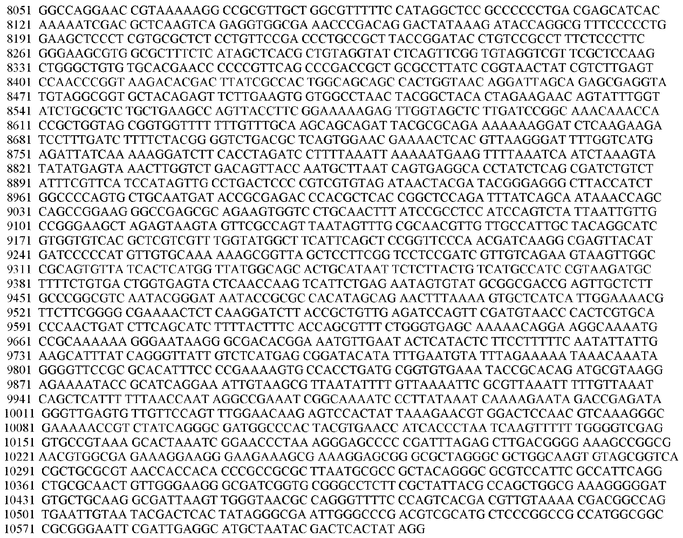

Figure 12 is a sequence listing of the DNA encoding infectious nucleic acid of CVA21-3'TR(2x) and the encoded RNA (SEQ ID NO:10). miRT(2x) is underlined.

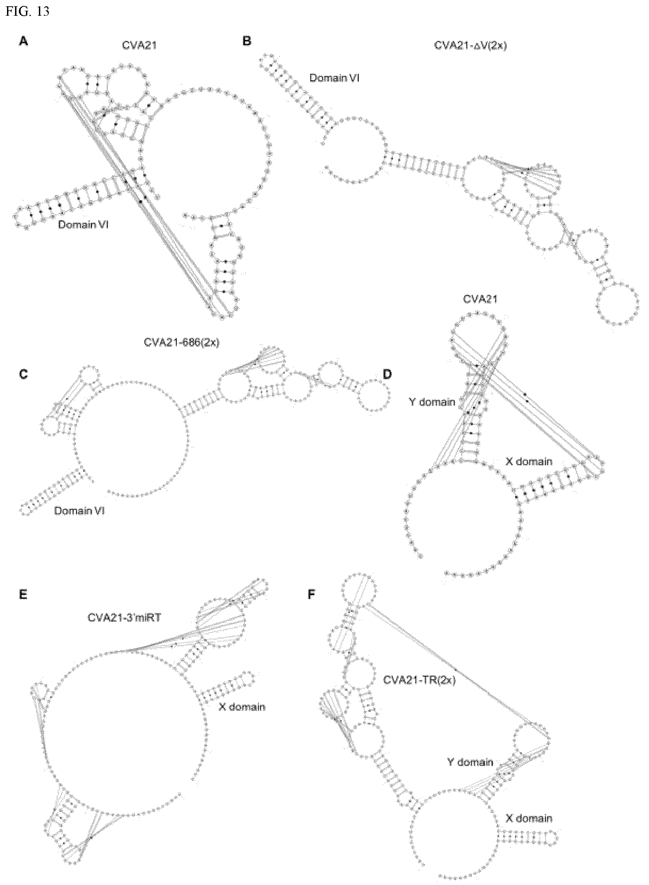

Figure 13. Predicted secondary RNA structures of microRNA-detargeted CVA21 non-coding regions versus unmodified. Predicted pseudoknot interactions are depicted by interloop lines. Secondary RNA structures were generated using the IPknot web server (rtips.dna.bio.keio.ac.jp/ipknot/) available through the Graduate School of Information Science, Nara Institute of Science and Technology Japan. 5' non-coding region predictions span domain VI and the scanning region and were predicted using level 2 (nested pseudoknots), CONTRAfold scoring model with refinements. 3' non-coding region predictions span the junction between *3D* and the entire 3'non-coding region with a 20 nucleotide long poly A tail and were predicted using level 3 (pseudoknotted with nested pseudoknots) prediction, CONTRAfold scoring model, with refinements. 5' non-coding region predictions include (A) unmodified CVA21; (B) CVA21-ΔV(2x); and (C) CVA21-686(2x). 3' non-coding region prediction for (D) CVA21; (E) CVA21-3'miRT; and (F) CVA21-TR(2x).

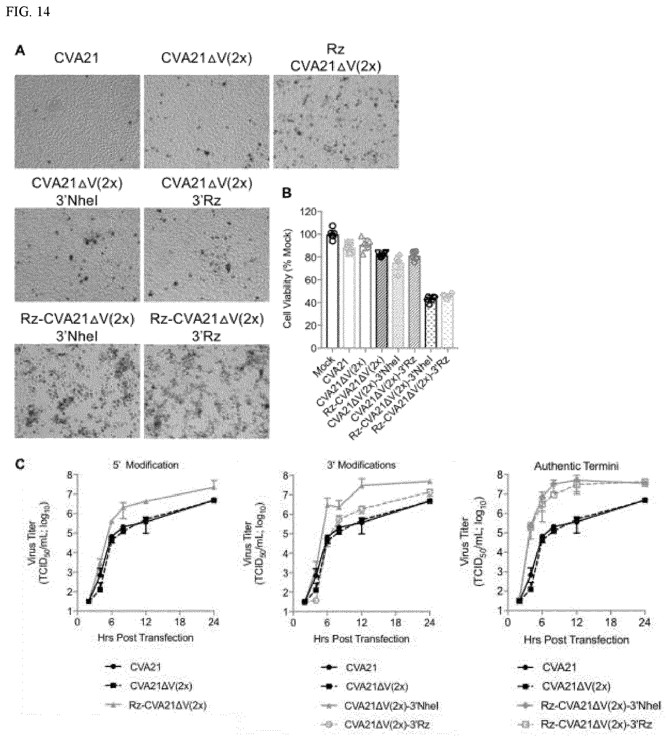

Figure 14. Authentic viral genome termini enhance virus recovery rate from *in* vitro-derived RNA transcripts encoding CVA21-ΔV(2x). (A) 1 x 10⁵ cells per well of H1-HeLa were transfected with 0.5 µg of *in vitro-*derived RNA transcripts encoding CVA21-ΔV(2x) with or without a ribozyme at the 5' end of the genome (Rz-CVA21-ΔV(2x)), either a restriction enzyme site (CVA21-ΔV(2x)-3'NheI) or a different ribozyme (CVA21-ΔV(2x)-3'Rz) at the 3' end directly adjacent to the poly A tail, or a combination of the 5' ribozyme and either the restriction enzyme site (Rz- CVA21-ΔV(2x)-3'NheI) or ribozyme (Rz- CVA21-ΔV(2x)-3'Rz) at the 3' end. 24 hours post transfection, the cells were stained with trypan blue and the CPE imaged. (B) The viability of cells treated as described in (A) was determined using a 3-(4,5-dimethylthiazolyl-2)-2,5-Diphenyltetrazolium bromide (MTT) kit (ATCC, Manassas, VA). (C) Time-course production of infectious virus in H1-HeLa cells transfected with 0.5 µg of infectious RNA encoding the constructs described in (A). The experiment was run in duplicate and data are represented as mean viral titers ± standard deviations.

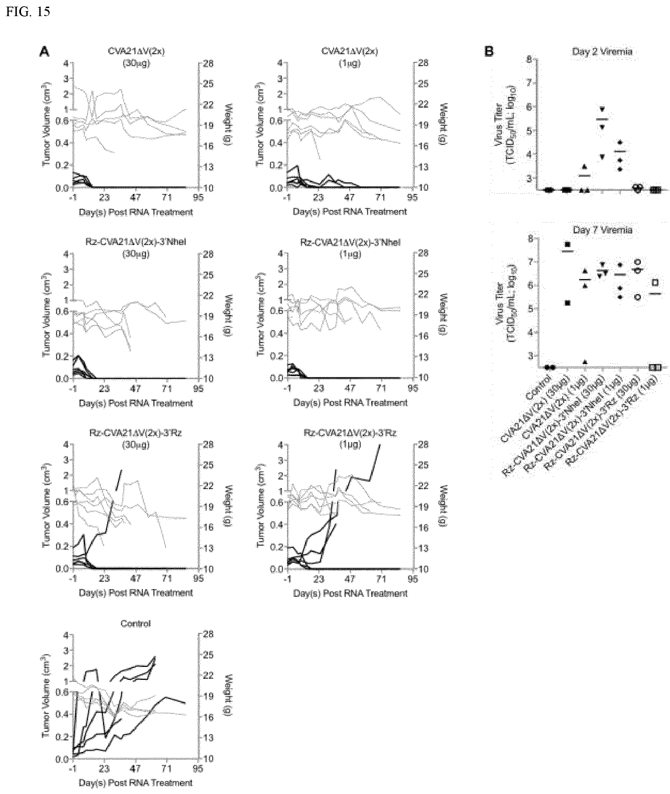

Figure 15. *In vitro*-derived RNA transcripts encoding CVA21-ΔV(2x) with authentic termini modifications mount a spreading oncolytic infection. CB-17 SCID mice bearing subcutaneous Mel624 xenografts were treated intratumorally with saline (*n* = 5), 30 µg (*n* = 5) or 1 µg (*n =* 6) CVA21-ΔV(2x), 30 µg (*n* = 6) or 1 µg (*n* = 6) Rz-CVA21-ΔV(2x)-3'NheI, or 30 µg (*n* = 6) or 1 µg of Rz-CVA21-ΔV(2x)-3'Rz RNA. (A) Tumor volumes (black) and weights (gray lines) of all treated mice. (B) Viral titers in sera collected on day 2 or day 7 post RNA administration from mice treated in A. All data points are distinct animals.

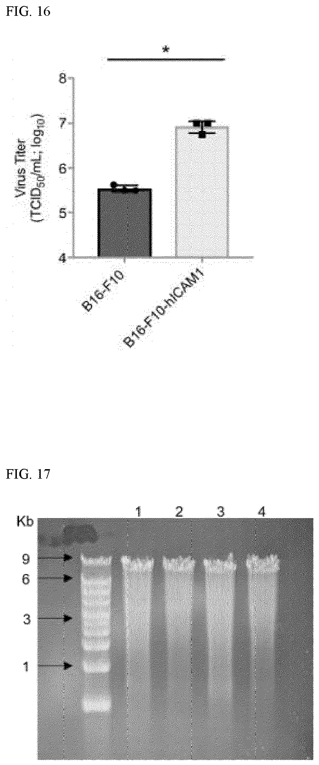

Figure 16. CVA21-ΔV(2x) can replicate in B16-F10 cells stably expressing human intracellular adhesion molecule 1 (hICAM-1), a receptor for CVA21. B16-F10-hICAM-1 cells or the parental B16-F10 cells were infected with CVA21-ΔV(2x) at an MOI of 1. 24 hours post infection, total virus titer was determined. CVA21-ΔV(2x) replication was significantly enhanced in cells expressing hICAM-1 compared to the parental cell line (*p* = 0.029). The experiment was run in triplicate, and data are represented as mean viral titers ± standard deviations.
Figure 17. CVA21-ΔV(2x) *in vitro* transcription reactions can be scaled to milligram levels and maintain similar integrity. Small scale (10 µl) and large scale (1 mL) reactions were run simultaneously and purified using lithium chloride precipitation. 1 µg of purified RNA from each reaction was run on an RNA FlashGel (Lonza). Lane 1. 10 µl reaction #1. Lane 2. 1 mL reaction #1. Lane 3. 10 µl reaction #2. Lane 4. 1 mL reaction #2. Total yields from each reaction were 150.55 µg; 7.1768 mg; 146.55 µg; and 7.3192 mg, respectively.

### DETAILED DESCRIPTION

This document provides methods and materials for using nucleic acid (e.g., infectious nucleic acid) encoding a virus (e.g., a picornavirus having a genome that includes a type I internal ribosome entry site) to reduce the number of viable cancer cells within a mammal. For example, this document provides methods for using infectious nucleic acid encoding a virus to reduce the number of viable cancer cells within a mammal. Nucleic acid provided herein can encode any appropriate virus and be used to reduce the number of viable cancer cells within a mammal. In some cases, a virus encoded by nucleic acid provided herein can replicate within a cancer cell. In some cases, infectious nucleic acid encoding a picornavirus can be used. A picornavirus can be an enterovirus (e.g., bovine enterovirus, human enterovirus A, human enterovirus B, human enterovirus C, human enterovirus D, human enterovirus E, poliovirus, porcine enterovirus A, and porcine enterovirus B), a rhinovirus (e.g., human rhinovirus A and human rhinovirus B), a cardiovirus (e.g., encephalomyocarditis virus and theilovirus), an apthovirus (e.g., equine rhinitis A virus and foot-and-mouth disease virus), an hepatovirus (e.g., hepatitis A virus), a parechovirus (e.g., human parechovirus and ljungan virus), an erbovirus (e.g., equine rhinitis B virus), a kobuvirus (e.g., aichi virus), or a teschovirus (e.g., porcine teschovirus 1-7 and porcine teschovirus). In some cases, infectious nucleic acid provided herein can encode a coxsackievirus A21 (Shafren et al., Clin. Cancer Res., 10(1 Pt. 1):53-60 (2004)), coxsackievirus B3 (Suskind et al., Proc. Soc. Exp. Biol. Med., 94(2):309-318 (1957)), poliovirus type III (Pond and Manuelidis, Am. J. Pathol., 45:233-249 (1964)), echovirus I (Shafren et al., Int. J. Cancer, 115(2):320-328 (2005)), or an encephalomyocarditis virus type E (Adachi et al., J. Neurooncol., 77(3):233-240 (2006)).

Nucleic acid (e.g., infectious nucleic acid) encoding a virus can be any appropriate nucleic acid (e.g., DNA, RNA, or a combination thereof). In some cases, nucleic acid encoding a virus can be a nucleic acid construct. For example, a nucleic acid construct encoding a virus can be plasmid DNA. For example, a nucleic acid construct encoding a virus can be an RNA molecule.

In some cases, nucleic acid (e.g., infectious nucleic acid) provided herein can encode a picornavirus having a genome that includes a type I internal ribosome entry site. Enteroviruses and rhinoviruses have type I IRESs. Examples of picornaviruses having a genome that includes a type I internal ribosome entry site include, without limitation, coxsackieviruses (e.g., coxsackievirus A21), polioviruses, echoviruses, enteroviruses (e.g., enterovirus-70), and rhinoviruses.

Nucleic acid (e.g., infectious nucleic acid) encoding a virus (e.g., a picornavirus such as a coxsackievirus A21) provided herein can be administered directly to cancer cells (e.g., by intratumoral administration) or can be administered systemically (e.g., by intravenous, intraperitoneal, intrapleural, or intra-arterial administration). The amount of infectious nucleic acid administered to a mammal can range from about 10 ng to about 1 mg (e.g., from 100 ng to 500 µg, from about 250 ng to about 250 µg, from about 500 ng to about 200 µg, or from about 1 µg to about 100 µg) per kg of body weight. In some cases, from about 100 ng to about 500 µg of infectious nucleic acid encoding a virus (e.g., a picornavirus such as a coxsackievirus A21) can be administered as a single intratumoral dose. In some cases, the amount of infectious nucleic acid administered to a mammal can be equal to a virus genome copy number of between about 3x10¹⁰ to about 3x10¹⁴ genome copies (e.g., between about 3x10¹⁰ to about 3x10¹³, between about 3x10¹⁰ to about 3x10¹², between about 3x10¹¹ to about 3x10¹⁴, between about 3x10¹¹ to about 3x10¹³, or between about 3x10¹¹ to about 3x10¹² genome copies). For example, infectious nucleic acid provided herein can be administered in an amount such that about 3x10¹¹ virus genome copies are delivered to a mammal. In some cases, the amount of administered infectious nucleic acid can be between about 3x10¹⁰ to about 3x10¹⁴ virus genome copies per kg of body weight.

Nucleic acid (e.g., infectious nucleic acid) encoding a virus (e.g., a picornavirus such as a coxsackievirus A21) can be designed to lack one or more (e.g., one, two, three, four, or more) contiguous nucleotide sequences of 10 or more nucleotides in length present in a wild-type version of that virus. For example, infectious nucleic acid encoding a virus (e.g., a picornavirus such as a coxsackievirus A21) can lack at least 10 (e.g., at least 10, 20, 30, 40, 50, 60, or more) contiguous nucleotides normally found between the VI domain of the 5' UTR and the translation start site (e.g., the AUG start site) for the viral polyprotein. When infectious nucleic acid encodes a coxsackievirus A21, the infectious nucleic acid can lack at least 10 (e.g., at least 10, 20, 30, 40, 50, 60, or more) contiguous nucleotides from position 631 to position 698 as found in the wild type coxsackievirus A21 genome. In some cases, infectious nucleic acid encoding a coxsackievirus A21 can be designed to lack all the nucleotides from position 631 to position 698 as found in the wild type coxsackievirus A21 genome.

As described herein, nucleic acid (e.g., infectious nucleic acid) encoding a virus (e.g., a picornavirus such as a coxsackievirus A21) can be designed to contain a microRNA target element such that a corresponding microRNA (miRNA, specific miRNAs denoted as miR-#) present within a non-cancer cell can reduce virus gene expression, virus replication, or virus stability in that non-cancer cell. MicroRNAs are small, 21-23 nucleotide, highly conserved regulatory RNAs that can mediate translational repression or, in some cases, mRNA destruction by RISC-induced cleavage. MicroRNAs are present within many mammalian cells and can have a tissue-specific tissue distribution. As such, microRNAs can be used to modulate the tropism of a replicating virus to provide a targeting approach for any virus. The ability of infectious nucleic acid encoding a virus (e.g., a picornavirus such as a coxsackievirus A21) to result in non-cancer cell lysis can be reduced using a microRNA target element having at least a region that is complementary to a microRNA present in the non-cancer cells. For example, wild-type coxsackievirus A21 can infect muscle cells. Thus, microRNA target elements that are complementary to microRNAs present in muscle cells can be incorporated into coxsackievirus A21 infectious nucleic acid to reduce muscle cell lysis. Similarly, the safety of vaccines can be improved by modulating the tropism of a virus. For example, a neuronal and/or brain microRNA target element can be incorporated into a poliovirus to reduce the incidence of poliomyelitis induced by an oral polio vaccine.

This same approach can be used to reduce non-cancer cell lysis by other viral nucleic acids. For example, microRNA target elements having at least a region that is complementary to the microRNAs set forth in Table 1 can be used to reduce cell lysis of the indicated tissue for the listed viruses as well as for other viruses. Other examples of microRNA target elements that can be designed to reduce viral-mediated cell lysis include, without limitation, those having at least a region complementary to a tissue-specific microRNA listed in Table 2. In some cases, infectious nucleic acid provided herein can encode a virus and contain a microRNA target element having at least a region complementary to a classified tissue-specific microRNA. MicroRNA target elements can have complete complementarity to a microRNA. In some cases, a microRNA target element can contain mismatches in its complementarity to a microRNA provided that it contains complete complementarity to a seed sequence (e.g., base pairs 2-7) of the microRNA. See, e.g., Lim et al., Nature, 433(7027):769-73 (2005)).

**Table 1. Silencing via incorporated microRNA target elements.**

| Virus | Tissue | microRNA |
|---|---|---|
| Coxsackievirus A21 | Muscle | miR-1 |
| Coxsackievirus A21 | Muscle | miR-133 |
| Coxsackievirus A21 | Muscle | miR-206 |
| Coxsackievirus B3, Encephalomyocarditis-E | Brain | miR-101 |
| Poliovirus III | | |
| Echovirus I | | |
| Coxsackievirus B3, Encephalomyocarditis-E | Brain | miR-124a,b |
| Poliovirus III | | |
| Echovirus I | | |
| Coxsackievirus B3, Encephalomyocarditis-E | Brain | miR-125 |
| Poliovirus III | | |
| Echovirus I | | |
| Coxsackievirus B3, Encephalomyocarditis-E | Brain | miR-128 |
| Poliovirus III | | |
| Echovirus I | | |
| Coxsackievirus B3, Encephalomyocarditis-E | Brain | miR-131 |
| Poliovirus III | | |
| Echovirus I | | |
| Coxsackievirus B3, Encephalomyocarditis-E | Brain | miR-132 |
| Poliovirus III | | |
| Echovirus I | | |
| Coxsackievirus B3, Encephalomyocarditis-E | Brain | miR-134 |
| Poliovirus III | | |
| Echovirus I | | |
| Coxsackievirus B3, Encephalomyocarditis-E | Brain | miR-135 |
| Poliovirus III | | |
| Echovirus I | | |
| Coxsackievirus B3, Encephalomyocarditis-E | Brain | miR-138 |
| Poliovirus III | | |
| Echovirus I | | |
| Coxsackievirus B3, Encephalomyocarditis-E | Brain | miR-153 |
| Poliovirus III | | |
| Echovirus I | | |
| Coxsackievirus B3, Encephalomyocarditis-E | Brain | miR-183 |
| Poliovirus III | | |
| Echovirus I | | |
| Coxsackievirus B3, Encephalomyocarditis-E | Brain | miR-1b-2 |
| Poliovirus III | | |
| Echovirus I | | |
| Coxsackievirus B3, Encephalomyocarditis-E | Brain | miR-219 |
| Poliovirus III | | |
| Echovirus I | | |
| Coxsackievirus B3, Encephalomyocarditis-E | Brain | miR-9 |
| Poliovirus III | | |
| Echovirus I | | |
| Coxsackievirus B3, Encephalomyocarditis-E | Brain | miR-95 |
| Poliovirus III | | |
| Echovirus I | | |
| Coxsackievirus B3, Encephalomyocarditis-E | Brain | miR-99b |
| Poliovirus III | | |
| Echovirus I | | |
| Coxsackievirus B3, Echovirus I | Heart | miR-1 |
| Coxsackievirus B3, Echovirus I | Heart | miR-133 |
| Coxsackievirus B3, Echovirus I | Heart | miR-206 |
| Coxsackievirus B3, Echovirus I | Heart | miR-208 |

**Table 2. Classified tissue-specific microRNAs.**

| **miRNA** | **Tissue** | **Sequence** | **Reference** |
|---|---|---|---|
| miR-1 | Muscle | UGGAAUGUAAAGAAGUAUGUA (SEQ ID NO:11) | Rao et al., Proc. Nat'l. Acad. Sci., 103:8721-8726 (2006). |
| miR-101 | Brain | UACAGUACUGUGAUAACUGAAG (SEQ ID NO:12) | Lagos-Quintana et al., Curr. Biol., 12:735-739 (2002). |
| miR-122a | Liver | UGGAGUGUGACAAUGGUGUUUGU (SEQ ID NO:13) | Fu et al., FEBS Lett., 579:3849-3854 (2005). |
| miR-124a,b | Brain | UUAAGGCACGCGGUGAAUGCCA (SEQ ID NO:14) | Lagos-Quintana et al., Curr. Biol., 12:735-739 (2002). |
| miR-125 | Brain | UCCCUGAGACCCUUUAACCUGUG (SEQ ID NO:15) | Liu et al., Proc. Nat'l. Acad. Sci., 101:9740-9744 (2004). |
| miR-126AS | Digestive | UCGUACCGUGAGUAAUAAUGC (SEQ ID NO:16) | Shingara et al., RNA, 11:1461-1470 (2005). |
| miR-127 | Spleen | UCGGAUCCGUCUGAGCUUGGCU (SEQ ID NO:17) | Lagos-Quintana et al., Curr. Biol., 12:735-739 (2002). |
| miR-128 | Brain | UCACAGUGAACCGGUCUCUUUC (SEQ ID NO:18) | Liu et al., Proc. Nat'l. Acad. Sci., 101:9740-9744 (2004). |
| miR-130 | Lung | CAGUGCAAUGUUAAAAGGGCAU (SEQ ID NO:19) | Sempere et al., Genome Biol., 5:R13 (2004). |
| miR-132 | Brain | UAACAGUCUACAGCCAUGGUCG (SEQ ID NO:20) | Lagos-Quintana et al., Curr. Biol., 12:735-739 (2002). |
| miR-133 | Muscle | UUGGUCCCCUUCAACCAGCUGU (SEQ ID NO:21) | Rao et al., Proc. Nat'l. Acad. Sci., 103:8721-8726 (2006). |
| miR-134 | Brain | UGUGACUGGUUGACCAGAGGG (SEQ ID NO:22) | Schratt et al., Nature, 439:283-289 (2006). |
| miR-135 | Brain | UAUGGCUUUUUAUUCCUAUGUGA (SEQ ID NO:23) | Sempere et al., Genome Biol., 5:R13 (2004). |
| miR-138 | Brain | AGCUGGUGUUGUGAAUC (SEQ ID NO:24) | Obemosterer et al., RNA, 12:1161-1167 (2006). |
| miR-142s 5p, 3p | Hematopoietic | CAUAAAGUAGAAAGCACUAC (SEQ ID NO:25) UGUAGUGUUUCCUACUUUAUGGA (SEQ ID NO:26) | Chen et al., Science, 303:83-86 (2004). |
| miR-143 | Digestive | UGAGAUGAAGCACUGUAGCUCA (SEQ ID NO:27) | Shingara et al., RNA, 11:1461-1470 (2005). |
| miR-145 | Digestive | GUCCAGUUUUCCCAGGAAUCCCUU (SEQ ID NO:28) | Shingara et al., RNA, 11:1461-1470 (2005). |
| miR-148 | Liver, Stomach | UCAGUGCACUACAGAACUUUGU (SEQ ID NO:29) | Shingara et al., RNA, 11:1461-1470 (2005). |
| miR-15 (Downregulated) | B-cell lymphocytic leukemia | UAGCAGCACAUAAUGGUUUGUG (SEQ ID NO:30) | Calin et al., Proc. Nat'l. Acad. Sci., 99:15524-15529 (2002). |
| miR-150 | Spleen | UCUCCCAACCCUUGUACCAGUG (SEQ ID NO:31) | Shingara et al., RNA, 11:1461-1470 (2005). |
| miR-151 | Spleen | ACUAGACUGAAGCUCCUUGAGG (SEQ ID NO:32) | Sempere et al., Genome Biol., 5:R13 (2004). |
| miR-152 | Liver | UCAGUGCAUGACAGAACUUGGG (SEQ ID NO:33) | Sempere et al., Genome Biol., 5:R13 (2004). |
| miR-153 | Brain | UUGCAUAGUCACAAAAGUGA (SEQ ID NO:34) | Sempere et al., Genome Biol., 5:R13 (2004). |
| miR-155 | Burkitt's Lymphoma | UUAAUGCUAAUCGUGAUAGGGG (SEQ ID NO:35) | Metzler et al., Genes Chromosomes Cancer, 39:167-169 (2004). |
| miR-16 (Downregulated) | B-cell lymphocytic leukemia | UAGCAGCACGUAAAUAUUGGCG (SEQ ID NO:36) | Calin et al., Proc. Nat'l. Acad. Sci., 99:15524-15529 (2002). |
| miR-17-5p | Lymphoma | CAAAGUGCUUACAGUGCAGGUAG U (SEQ ID NO:37) | He et al., Nature, 435:828-833 (2005). |
| miR-181 | Hematopoietic | AACAUUCAACGCUGUCGGUGAGU (SEQ ID NO:38) | Chen et al., Science, 303:83-86 (2004). |
| miR-183 | Brain | UAUGGCACUGGUAGAAUUCACUG (SEQ ID NO:39) | Sempere et al., Genome Biol., 5:R13 (2004). |
| miR-18a,b | Lymphoma | UAAGGUGCAUCUAGUGCAGAUA (SEQ ID NO:40) | He et al., Nature, 435:828-833 (2005). |
| | | UAAGGUGCAUCUAGUGCAGUUA (SEQ ID NO:41) | |
| miR-192 | Kidney | CUGACCUAUGAAUUGACAGCC (SEQ ID NO:42) | Sempere et al., Genome Biol., 5:R13 (2004). |
| miR-194 | Kidney | UGUAACAGCAACUCCAUGUGGA (SEQ ID NO:43) | Sun et al., Nucleic Acids Res., 32:e188 (2004). |
| miR-195 | Hematopoietic | UAGCAGCACAGAAAUAUUGGC (SEQ ID NO:44) | Baskerville et al., RNA, 11:241-247 (2005). |
| miR-199 | Liver | CCCAGUGUUCAGACUACCUGUUC (SEQ ID NO:45) | Sempere et al., Genome Biol., 5:R13 (2004). |
| miR-19a,b | Lymphoma | UGUGCAAAUCUAUGCAAAACUGA (SEQ ID NO:46) | He et al., Nature, 435:828-833 (2005). |
| | | UGUGCAAAUCCAUGCAAAACUGA (SEQ ID NO:47) | |
| miR-204 | Kidney | UUCCCUUUGUCAUCCUAUGCCU (SEQ ID NO:48) | Sun et al., Nucleic Acids Res., 32:e188 (2004). |
| miR-204 | Testis | UUCCCUUUGUCAUCCUAUGCCU (SEQ ID NO:49) | Baskerville et al., RNA, 11:241-247 (2005). |
| miR-206 | Muscle | UGGAAUGUAAGGAAGUGUGUGG (SEQ ID NO:50) | Rao et al., Proc. Nat'l. Acad. Sci., 103:8721-8726 (2006). |
| miR-208 | Heart | AUAAGACGAGCAAAAAGCUUGU (SEQ ID NO:51) | Sempere et al., Genome Biol., 5:R13 (2004). |
| miR-212 | Spleen | UAACAGUCUCCAGUCACGGCC (SEQ ID NO:52) | Sempere et al., Genome Biol., 5:R13 (2004). |
| miR-215 | Liver | AUGACCUAUGAAUUGACAGAC (SEQ ID NO:53) | Sempere et al., Genome Biol., 5:R13 (2004). |
| miR-215 | Kidney | AUGACCUAUGAAUUGACAGAC (SEQ ID NO:54) | Sun et al., Nucleic Acids Res., 32:e188 (2004). |
| miR-216 | Pancreas | UAAUCUCAGCUGGCAACUGUG (SEQ ID NO:55) | Sood et al., Proc. Nat'l. Acad. Sci., 103:2746-2751 (2006). |
| miR-219 | Brain | UGAUUGUCCAAACGCAAUUCU (SEQ ID NO:56) | Sempere et al., Genome Biol., 5:R13 (2004). |
| miR-221 | Hematopoietic | AGCUACAUUGUCUGCUGGGUUUC (SEQ ID NO:57) | Felli et al., Proc. Nat'l. Acad. Sci., 102:18081 18086 (2005). |
| miR-222 | Hematopoietic | AGCUACAUCUGGCUACUGGGUCUC (SEQ ID NO:58) | Felli et al., Proc. Nat'l. Acad. Sci., 102:18081 18086 (2005). |
| miR-223 | Hematopoietic | UGUCAGUUUGUCAAAUACCCC (SEQ ID NO:59) | Chen et al., Science, 303:83-86 (2004). |
| miR-24 | Lung | UGGCUCAGUUCAGCAGGAACAG (SEQ ID NO:60) | Sempere et al., Genome Biol., 5:R13 (2004). |
| miR-25 | Lymphoma | CAUUGCACUUGUCUCGGUCUGA (SEQ ID NO:61) | He et al., Nature, 435:828-833 (2005). |
| miR-30b,c | Kidney | UGUAAACAUCCUACACUCAGCU (SEQ ID NO:62) | Sempere et al., Genome Biol., 5:R13 (2004). |
| | | UGUAAACAUCCUACACUCUCAGC (SEQ ID NO:63) | |
| miR-32 | Lung | UAUUGCACAUUACUAAGUUGC (SEQ ID NO:64) | Sempere et al., Genome Biol., 5:R13 (2004). |
| miR-375 | Pancreas | UUUGUUCGUUCGGCUCGCGUGA (SEQ ID NO:65) | Poy et al., Nature, 432:226-230 (2004). |
| miR-7 | Pituitary | UGGAAGACUAGUGAUUUUGUUG (SEQ ID NO:66) | He et al., Nature, 435:828-833 (2005). |
| miR-9 | Brain | UCUUUGGUUAUCUAGCUGUAUGA (SEQ ID NO:67) | Sun et al., Nucleic Acids Res., 32:e188 (2004). |
| miR-95 | Brain | UUCAACGGGUAUUUAUUGAGCA (SEQ ID NO:68) | Babak et al., RNA, 10:1813-1819 (2004). |
| miR-99b | Brain | CACCCGUAGAACCGACCUUGCG (SEQ ID NO:69) | Liu et al., Proc. Nat'l. Acad. Sci., 101:9740-9744 (2004). |

Molecular cloning techniques can be used to insert microRNA target elements into nucleic acid (e.g., infectious nucleic acid) encoding a virus (e.g., a picornavirus such as a coxsackievirus A21). An infectious nucleic acid provided herein can contain one microRNA target element or multiple microRNA target elements (e.g., two, three, four, five, six, seven, eight, nine, ten, 15, 20, 25, 30, or more microRNA target elements). For example, an infectious nucleic acid provided herein can include two different microRNA target elements such as one that is a target of miR-133 and one that is a target of miR-206. In some cases, an infectious nucleic acid provided herein can include two or more identical microRNA target elements. For example, an infectious nucleic acid provided herein can include two microRNA target elements that each are a target of miR-133 or two microRNA target elements that each are a target of miR-206. In some cases, an infectious nucleic acid provided herein can include two or more (e.g., two, three, four, or more) microRNA target elements that each are a target of miR-133 and two or more (e.g., two, three, four, or more) microRNA target elements that each are a target of miR-206.

As described herein, one or more (e.g., one, two, three, four, five, six, or more) microRNA target elements can be inserted into nucleic acid (e.g., infectious nucleic acid) encoding a virus (e.g., a picornavirus such as a coxsackievirus A21) between the VI domain of the 5' UTR and the translation start site (e.g., the AUG start site) for the viral polyprotein. When infectious nucleic acid encodes a coxsackievirus A21, the infectious nucleic acid can include one or more (e.g., one, two, three, four, five, six, or more) microRNA target elements inserted between position 631 and position 698 as found in the wild type coxsackievirus A21 genome. In some cases, an infectious nucleic acid encoding a virus (e.g., a picornavirus such as a coxsackievirus A21) provided herein can lack at least 10 (e.g., at least 10, 20, 30, 40, 50, 60, or more) contiguous nucleotides normally found between the VI domain of the 5' UTR and the translation start site (e.g., the AUG start site) for the viral polyprotein and can include within this same location one or more (e.g., one, two, three, four, five, six, or more) microRNA target elements. In some cases, infectious nucleic acid encoding a coxsackievirus A21 can be designed to lack all the nucleotides from position 631 to position 698 as found in a wild type coxsackievirus A21 genome (e.g., the Kuykendall CVA21 strain) and can include, in place of those removed nucleotides, one or more (e.g., one, two, three, four, five, six, or more) microRNA target elements. Examples of such infectious nucleic acid are set forth in Figures 8 and 9. Other examples of infectious nucleic acid provided herein are set forth in Figures 10 and 11.

In some cases, microRNA target elements that are complementary to microRNAs that are ubiquitously expressed in normal cells with limited expression in cancer cells can be used to direct cell lysis to cancer cells and not non-cancer cells. For example, when using nucleic acid coding for a virus to treat B-cell lymphocytic leukemia, the nucleic acid (e.g., infectious nucleic acid) can be designed to contain microRNA target elements complementary to microRNAs that are ubiquitously expressed in normal tissue while being downregulated in B-cell lymphocytic leukemia cells. Examples of such microRNAs include, without limitation, miR-15 and miR-16.

Nucleic acid (e.g., infectious nucleic acid) encoding a virus (e.g., a picornavirus such as a coxsackievirus A21) can be designed to include a ribozyme or nucleic acid encoding a ribozyme. For example, in some cases, an infectious RNA encoding a virus (e.g., a picornavirus such as a coxsackievirus A21) can be designed to include a ribozyme designed to cleave a portion of RNA from itself, and in some cases, an infectious DNA encoding a virus (e.g., a picornavirus such as a coxsackievirus A21) can be designed to include a DNA sequence encoding a ribozyme designed to cleave a portion of RNA from the RNA molecule transcribed from the infectious DNA. Such a ribozyme can be a hammerhead ribozyme, a hepatitis delta virus ribozyme, a hairpin ribozyme, a Varkud Satellite ribozyme, a glmS ribozyme, a Twister ribozyme, a Twister sister ribozyme, a Hatchet ribozyme, a Pistol ribozyme, or a synthetic ribozyme. A ribozyme can be designed to remove any portion of RNA from an infectious RNA. For example, a ribozyme can be designed to remove a 5' end portion or a 3' end portion of RNA from an infectious RNA encoding a virus. In some case, infectious nucleic acid (DNA or RNA) encoding a virus (e.g., a picornavirus such as a coxsackievirus A21) can be designed to include (a) either nucleic acid encoding a first ribozyme in the case of DNA or a first ribozyme in the case of RNA and (b) either nucleic acid encoding a second ribozyme in the case of DNA or a second ribozyme in the case of RNA. In such cases, the first ribozyme can be designed to remove a 5' end portion of RNA from an infectious RNA encoding a virus and the second ribozyme can be designed to remove a 3' end portion. In some cases, after restriction endonuclease cleavage of nucleic acid encoding a virus, a virus encoded by the nucleic acid encoding a virus can include viral-encoding sequences with less than 88 (e.g., less than 63, or less than 10 such as 5, 4, 3, 2, 1, or 0) non-viral nucleotides (e.g., near authentic termini). In some cases, the resulting infectious RNA encoding a virus after ribozyme cleavage can include viral-encoding sequences with no non-viral sequences (e.g., authentic termini).

Nucleic acid (e.g., infectious nucleic acid) encoding a virus (e.g., a picornavirus such as a coxsackievirus A21) can be designed to include a restriction endonuclease cut site. For example, nucleic acid (e.g., DNA) encoding a virus (e.g., a picornavirus such as a coxsackievirus A21) can be designed to include a restriction endonuclease cut site designed to cleave a portion of nucleic acid from the infectious nucleic acid. In some cases, DNA (e.g., infectious DNA) encoding a virus (e.g., a picornavirus such as a coxsackievirus A21) can be designed to include a DNA sequence such that the infectious DNA includes a restriction endonuclease cut site capable of being cleaved by a restriction endonuclease that cuts RNA at that restriction endonuclease cut site. In some cases, RNA (e.g., infectious RNA) encoding a virus (e.g., a picornavirus such as a coxsackievirus A21) can be designed to include a restriction endonuclease cut site capable of being cleaved by a restriction endonuclease that cuts RNA and/or DNA at that restriction endonuclease cut site. Any appropriate restriction endonuclease cut site and restriction endonuclease capable of cleaving nucleic acid at that restriction endonuclease cut site can be used. Examples of restriction endonuclease cut sites and restriction endonuclease capable of cleaving nucleic acid at those restriction endonuclease cut sites are provided in Table 3.

**Table 3. Restriction endonuclease/cut site pairing. R is a purine (A or G). Y is a pyrimidine (A or T).**

| **Restriction endonuclease** | **Cut site** |
|---|---|
| NsiI | ATGCA^T |
| BmtI | GCTAG^C |
| FseI | GGCCGG^CC |
| AsiSI | GCGAT^CGC |
| BstBI | TT^CGAA |
| NheI | G^CTAGC |
| MluI | A^CGCGT |
| HaeII | RGCGC^Y |
| NspI | RCATG^Y |

A restriction endonuclease cut site can be designed such that cleavage at that cut site removes any portion of nucleic acid from nucleic acid (e.g., infectious nucleic acid) encoding a virus (e.g., a picornavirus such as a coxsackievirus A21). For example, a restriction endonuclease cut site can be positioned to remove a 5' end portion and/or a 3' end portion of nucleic acid from nucleic acid encoding a virus. In some case, infectious nucleic acid encoding a virus (e.g., a picornavirus such as a coxsackievirus A21) can be designed to include one, two, three, four, or more restriction endonuclease cut sites. For example, a first restriction endonuclease cut site can be positioned to remove a 5' end portion of RNA from an infectious RNA encoding a virus and a second endonuclease cut site can be positioned to remove a 3' end portion. When including two or more restriction endonuclease cut sites, the cut sites can be the same (e.g., two NheI cut sites) such that the same restriction endonuclease (e.g., NheI) cuts those sites, or the cut sites can be different (e.g., one NheI cut site and one cut site that is not an NheI cut site) such that different restriction endonucleases (e.g., NheI and one cut site that is not an NheI cut site) cut the different sites. In some cases, after restriction endonuclease cleavage of nucleic acid encoding a virus, a virus encoded by the nucleic acid encoding a virus can include viral-encoding sequences with less than 88 (e.g., less than 63, or less than 10 such as 5, 4, 3, 2, 1, or 0) non-viral nucleotides (e.g., near authentic termini). In some cases, the resulting infectious RNA encoding a virus after restriction endonuclease cleavage can include viral-encoding sequences with no non-viral sequences (e.g., authentic termini).

In some cases, nucleic acid (e.g., infectious nucleic acid) encoding a virus (e.g., a picornavirus such as a coxsackievirus A21) can be designed to include a combination of one or more ribozymes in the case of infectious RNA (or a combination of sequences encoding one or more ribozymes and one or more restriction endonuclease cut sites in the case of infectious DNA). For example, nucleic acid encoding a virus can include a DNA sequence encoding a ribozyme designed to remove a 5' end portion of the encoded virus, and can include a restriction endonuclease cut site designed to be cleaved by a restriction endonuclease to remove a 3' end portion of the nucleic acid such that encoded virus contains no non-viral sequences (e.g. authentic termini).

In some cases, when using restriction endonuclease cut site(s), the restriction endonuclease(s) capable of cutting that restriction endonuclease cut site(s) can be exogenously added to a solution containing the nucleic acid (e.g., infectious nucleic acid such as infectious RNA) to be cleaved. For example, an exogenously added restriction endonuclease can be added to a solution containing the infectious nucleic acid to be cleaved under conditions that allow the restriction endonuclease to cleave the infectious nucleic acid. In some cases, a solution that includes a restriction endonuclease and infectious nucleic acid to be cleaved can be incubated for about 60 minutes to about 300 minutes (e.g., for about 60 minutes to about 240 minutes, for about 60 minutes to about 200 minutes, for about 60 minutes to about 180 minutes, for about 60 minutes to about 150 minutes, for about 60 minutes to about 120 minutes, for about 60 minutes to about 100 minutes, for about 60 minutes to about 90 minutes, for about 90 minutes to about 300 minutes, for about 100 minutes to about 300 minutes, for about 120 minutes to about 300 minutes, for about 150 minutes to about 300 minutes, for about 180 minutes to about 300 minutes, for about 200 minutes to about 300 minutes, for about 240 minutes to about 300 minutes, for about 90 minutes to about 240 minutes, for about 120 minutes to about 210 minutes, for about 150 minutes to about 180 minutes, for about 60 minutes to about 90 minutes, for about 90 minutes to about 120 minutes, for about 120 minutes to about 150 minutes, for about 150 minutes to about 180 minutes, for about 180 minutes to about 210 minutes, for about 210 minutes to about 240 minutes, or for about 240 minutes to about 270 minutes) to allow the restriction endonuclease to cleave the infectious nucleic acid. In some cases, a solution that includes a restriction endonuclease and infectious nucleic acid to be cleaved can be incubated at about 37 °C to about 60 °C (e.g., at about 37 °C, at about 42 °C, at about 45 °C, at about 50°C, or at about 55 °C) to allow the restriction endonuclease to cleave the infectious nucleic acid. For example, when a restriction endonuclease is NheI, a solution that includes NheI and infectious nucleic acid to be cleaved can be incubated for about 60 minutes to about 180 minutes at about 37 °C to allow the NheI to cleave the infectious nucleic acid. In some cases, after incubating a solution containing an exogenously added restriction endonuclease and an infectious nucleic acid to be cleaved under conditions that allow the restriction endonuclease to cleave the infectious nucleic acid, the cleaved infectious nucleic acid can be isolated from the solution. Any appropriate technique can be used to isolate cleaved infectious nucleic acid from the solution. For example, ethanol precipitation or column purification can be used to isolate cleaved infectious nucleic acid from the solution.

When nucleic acid (e.g., infectious nucleic acid) encoding a virus (e.g., a picornavirus such as a coxsackievirus A21) are administered to a mammal to treat cancer (e.g., B-cell lymphocytic leukemia), the mammal also can be administered one or more additional cancer treatments. The one or more additional cancer treatments can include any appropriate cancer treatment(s). In some cases, a cancer treatment can include surgery. In some cases, a cancer treatment can include radiation therapy. In some cases, a cancer treatment can include administration of one or more anti-cancer agents such as a chemotherapeutics, checkpoint inhibitors (e.g., CTLA-4 inhibitors, PD-1 inhibitors, and PD-L1 inhibitors), oncolytic viruses, gene therapies, histone deacetylase (HDAC) inhibitors, antimicrobials, immunotherapies, vaccines, protein kinase inhibitors, second mitochondrial-derived activator of caspases (SMAC) mimetics, and/or holistic therapies. An anti-cancer agent can be any appropriate type of molecule (e.g., a polypeptide such as an antibody or a small molecule). Examples of anti-cancer agents include, without limitation, ipilimumab, nivolumab, pembrolizumab, atezolizumab, avelumab, durvalumab, cemiplimab, spartalizuma, talimogene laherparepvec (T-vec), trichostatin A (TSA), panobinostat, entinostat, romidepsin, vorinostat ,givinostat, adavosertib, afatinib, axitinib, bosutinib, cetuximab, cobimetinib, crizotinib, cabozantinib, dasatinib, entrectinib, erdafitinib, erlotinib, fostamatinib, gefitinib, ibrutinib, imatinib, lapatinib, lenvatinib, mubritinib, nilotinib, pazopanib, pegaptanib, ruxolitinib, sorafenib, sunitinib, vandetanib, vemurafenib, and combinations thereof. For example, a mammal having cancer (e.g., B-cell lymphocytic leukemia) can be treated by administering nucleic acid encoding a picornavirus such as a coxsackievirus A21 and administering one or more checkpoint inhibitors (e.g., CTLA-4 inhibitors, PD-1 inhibitors, and/or PD-L1 inhibitors) to the mammal.

In cases where a mammal having cancer is treated with nucleic acid (e.g., infectious nucleic acid) encoding a virus (e.g., a picornavirus such as a coxsackievirus A21), and is treated with one or more cancer treatments (e.g., is administered one or more anti-cancer agents), the cancer treatment(s) can be administered at the same time or independently. For example, the nucleic acid encoding a virus can be administered first, and the one or more cancer treatments administered second, or vice versa.

Also provided herein are immunocompetent models that can be infected by nucleic acid (e.g., infectious nucleic acid) encoding a virus (e.g., a picornavirus such as a coxsackievirus A21). For example, cells expressing a virus receptor (e.g., ICAM-1) can be infected by infectious nucleic acid encoding a virus (e.g., a picornavirus such as a coxsackievirus A21). When a virus receptor is ICAM-1, the ICAM-1 can be from any source. For example, an ICAM-1 can be a human ICAM-1. In some cases, an immunocompetent model does not endogenously express a virus receptor. An immunocompetent model expressing a virus receptor can stably express the virus receptor or can transiently express the virus receptor. In some cases, an immunocompetent model described herein can be used to replicate the virus encoded by nucleic acid encoding a virus. In some cases, an immunocompetent model described herein can be used as a model to evaluate and/or monitor the specificity of infection (e.g., following virus production).

In some cases, an immunocompetent model that can be infected by nucleic acid (e.g., infectious nucleic acid) encoding a virus can be a cell (e.g., a cell line) that can express (e.g., are designed to express) a virus receptor (e.g., ICAM-1 such as human ICAM-1). Any appropriate cell can be used to make an immunocompetent model described herein. In some cases, a cell used to make an immunocompetent model described herein can be obtained from a mammal (e.g., can be a primary cell). In some cases, a cell used to make an immunocompetent model described herein can be obtained from a cell line (e.g., a mammalian cell line such as murine melanoma B16-F10 cells).

In some cases, an immunocompetent model that can be infected by nucleic acid (e.g., infectious nucleic acid) encoding a virus can be a non-human animal model (e.g., a mouse model) having one or more cells that can express (e.g., are designed to express) a virus receptor (e.g., ICAM-1). Any appropriate non-human animal can be used to make an immunocompetent model described herein. In some cases, a non-human animal used to make an immunocompetent model described herein can be a mammal (e.g., a mouse or a rat).

Any appropriate method can be used to make an immunocompetent model described herein. When an immunocompetent model is a cell, nucleic acid encoding a virus receptor can be introduced into a cell such that the virus receptor is expressed by the cell. For example, a lentiviral vector encoding a virus receptor can be transduced into a cell such that the virus receptor is expressed by the cell. When an immunocompetent model is a non-human animal, nucleic acid encoding a virus receptor can be introduced into one or more cells within the non-human animal such that the virus receptor is expressed by one or more cells within the non-human animal. The invention will be further described in the following examples, which do not limit the scope of the invention described in the claims.

### EXAMPLES

### Example 1 - Generating infectious nucleic acid that can be used to treat cancer

Enteroviruses and rhinoviruses use a type I internal ribosome entry site (IRES) for regulating viral translation. The structure of the poliovirus IRES was predicted and validated via digestion/chemical probing and mutagenic analysis (Rivera et al., Virology, 165:42-50 (1988); Pilipenko et al., Virology, 168:201-209 (1989); Skinner et al., J. Mol. Biol., 207:379-392 (1989); and Burrill et al., J. Virol., 87:11670-11683 (2013)). CVA21 is a polio-like virus and shares relatively 86% homology with the poliovirus IRES. The poliovirus 5' UTR contains six domains (I to VI). Domain I forms a cloverleaf structure that is required for both positive and minus-strand synthesis (Andino et al., Cell, 63:369-380 (1990); Andino et al., EMBO J., 12:3587-3598 (1993); Barton et al., EMBO J., 20:1439-1448 (2001); and Vogt et al., PLoS Pathog., 6:e1000936 (2010)). Domains II thru VI are involved in IRES activity. Viral replication and translation require complex RNA-RNA and RNA-protein interactions including recruitment of host *IRES-trans* acting factors (ITAFs) and initiation factors (Lee et al., Trends Microbiol., 25:546-561 (2017)). Disruption of these domains can result in attenuation or lethal phenotypes for the virus. Similar to poliovirus, CVA21 has a cryptic AUG site in domain VI involved in ribosome loading (Verma et al., J. Gen. Virol., 92:2310-2319 (2011)). Initiation of translation at the primary AUG site depends upon ribosome scanning through a long variable linker referred to as the "scanning region."

Infectious nucleic acid was designed to include a microRNA response element (RE) positioned within this scanning region. The RE was designed to lack AUG sites that may initiate translation prior to the authentic AUG.

Lethal myositis was the primary toxicity that needed to be eliminated to elevate the safety profile of CVA21 to a level sufficient for treatment in immunocompromised patients. The REs provided herein were directed towards eliminating viral replication within muscle tissues. miR-133 and miR-206 were selected. They are highly enriched within muscle tissues and were previously shown to eliminate toxicity when inserted into the 3' UTR of CVA21 as described elsewhere (Kelly et al., Nat. Med., 14:1278-1283 (2008)).

A single microRNA-target is sufficient to down-regulate viral replication, however, there are a significant number of variables that impact the efficiency of targeting. Additionally, RNA viruses can accumulate mutations rapidly due to the low fidelity of the RdRp, which can result in loss of RE functionality shortly after infection. Therefore, REs were designed to encode either one copy each (miRT-1x) or two copies each (miRT-2x) of sequences complementary to miR-133 and miR-206. The sequences for the REs are shown in Figure 1A. AscI sites were inserted into pGEM-CVA21 at the desired locations for REs either by overlap-extension PCR, site-directed mutagenesis or by synthesizing fragments of the genome followed by subcloning into the full-length construct (pGEM-CVA21). All REs were inserted into the viral genome by annealing oligonucleotide ultramers encoding the RE flanked by the overhang sequences generated during an AscI enzymatic digestion followed by ligation into the appropriately digested and purified AscI-full-length vectors.

Three different constructs were generated encoding REs in the 5' UTR. The first involved inserting a 2x-RE into the scanning region at nucleotide position 686 (CVA21-686(2x)). In addition, two constructs were generated where residues 631 thru 698 within the variable scanning region were deleted and the REs were added. The first contained a miRT-1x RE (CVA21-ΔV(1x)), and the other contained a miRT-2x RE (CVA21-ΔV(2x)). Figure 1B depicts the CVA21 viral genome, the structural elements of the UTRs, and the RE insertion sites tested.

The majority of previously tested cellular microRNA target sequences are located within the 3' UTR of the targeted mRNAs. Based on experimentally verified modeling of the poliovirus 3' UTR (Pilipenko et al., EMBO J., 15:5428-5436 (1996)), the secondary structure of the 3' UTR of CVA21 (a polio-like virus) was thought to contain two hairpins (Y and X) whose loops form a pseudoknot interaction known as a "kissing domain" (van Ooij et al., J. Gen. Virol., 87:689-695 (2006)). These structures included the origin of replication (oriR) for minus-strand synthesis. Destabilization of the kissing domain in other enteroviruses severely inhibited viral RNA synthesis, resulting in temperature sensitive mutants or lethal phenotypes (van Ooij et al., J. Gen. Virol., 87:689-695 (2006); Melchers et al., J. Virol., 71:686-696 (1997); Wang et al., Nucleic Acids Res., 27:485-490 (1999); and Melchers et al., RNA, 6:976-987 (2000)). In poliovirus and coxsackievirus B3, serial passage of the destabilized viruses resulted in revertants wherein the pseudoknot interaction was restored (Pilipenko et al., EMBO J., 15:5428-5436 (1996); and van Ooij et al., J. Gen. Virol., 87:689-695 (2006)). Deletion of the two domains also resulted in truncation of the poly(A) tail (van Ooij et al., Nucleic Acids Res., 34:2953-2965 (2006)). Viruses were recovered from these deletion mutants, however, they acquired poly (AU) stretches corresponding to cellular polyadenylation signals. The lengths of the X and Y domains were highly conserved among enteroviruses, and it was shown that domain Y should be 12 bp in combination with an 8 bp stem for domain X. Additionally, the two most distal base pairs in domain Y relative to the "kissing domain" were required to be Watson-Crick CG pairs (Melchers et al., J. Virol., 71:686-696 (1997)). The length of the linker regions between the two stem loops was important for the correct orientation of the helices and their ability to interact. Finally, the existence of an S domain was also suggested wherein the poly(A) tail base pairs with a tract of four uridine residues upstream of the stop codon in the *3D* gene. This domain closed off the oriR structure, creating a more rigid 3' UTR structure (Pilipenko et al., Nucleic Acids Res., 20:1739-1745 (1992)). Having a properly folded 3' UTR was therefore involved to ensure correct docking and orientation of the proteins associated with the ribonucleoprotein complex involved in negative-sense strand synthesis.

The left panel in Figure 1C shows the secondary structure model of the CVA21 *oriR* and the location of the RE used in the Kelly *et al.* construct (CVA21-3'miRT) at nucleotide position 7343. Insertion at this site disrupted the length of domain Y, interfered with the distal CG base pairs, and likely disrupted the linker region between domains X and Y, interfering with the orientation of the helices. All residues in the 3' UTR and several in the coding sequence were involved in maintaining the *oriR* structure, limiting potential insertion sites for REs. To bypass any structural disruptions, an AscI restriction site followed by a terminal repeat (TR) element was inserted directly downstream of the stop codon. This TR element included nucleotides 7325 to 7340, containing the four uridine residues thought to be involved in domain S. It was hypothesized that the TR would separate the oriR structure from the RE inserted into the AscI site while maintaining the coding sequence (Figure 1C, right). Both miRT-1x (CVA21-3'TR(1x)) and miRT-2x (CVA21-3'TR(2x)) containing constructs were generated.

MicroRNA target insert integrity was verified in all constructs by sequencing the insert region.

### Virus Rescue Kinetics from RNA Transcripts

One objective was to obtain infectious nucleic acid that can nucleate a spreading virus infection from targeted-infectious nucleic acid at a rate similar to the unmodified virus. To analyze the ability of the targeted infectious RNAs to produce virus progeny H1-HeLa cells (ATCC, Manassas, VA) that do not express miR-133 or miR-206 were transfected with infectious RNA encoding each miRT-CVA21. Infectious RNA was prepared by linearizing plasmid DNA encoding unmodified or miRT-CVA21 with restriction endonuclease MluI-HF (New England Biolabs, Ipswich, MA), followed by ethanol precipitation and resuspension in nuclease-free water. 1 µg of linearized DNA was transcribed into RNA transcripts using the Ambion MEGAscript T7 transcription kit, and the RNA was purified using the MEGAclear transcription clean-up kit (Thermo Fisher Scientific Inc., Waltham, MA); both according to the manufacturers' instructions. Transcript size and integrity were verified by running the RNA on an RNA Flash gel (Lonza, Basel, Switzerland). 4 x 10⁵ cells were seeded per well in 6-well tissue culture plates, 24 hours prior to transfection. Each well was transfected with 2.5 µg of purified T7 RNA using *TransIT-mRNA* transfection kit (Mirus Bio LLC, Madison, WI). Once cytopathic effects (CPE) were observed, the cells were scraped into the supernatant, and the samples collected into individual cryotubes. The samples were subjected to 3 freeze-thaw cycles, cleared by centrifugation at 2500 rpm for 5 minutes at 4 °C, and filtered through a 0.22 µm syringe filter. 50 µL of cleared lysate was used to infect fresh H1-HeLa cells in a well of a 6-well tissue culture plate. Cells were infected for 2 hours at 37 °C in serum-free media. Media and unincorporated virus were removed, and 2 mL of fresh complete growth media were added per well. At 24 hours post infection, the cells were observed for CPE. All miRT-CVA21 except the Kelly *et al.* construct, CVA21-3'miRT, generated sufficient virus progeny to produce visible CPE (Figure 2A).

To evaluate the rate at which virus progeny were generated from RNA transcripts encoding these targeted viral genomes, growth curve time courses were conducted in H1-HeLa cells. 2.5 x 10⁵ H1-HeLa cells were seeded per well into 12-well tissue culture dishes, 24 hours prior to transfection. Each well was transfected with 1 µg of purified T7 RNA using *TransIT-mRNA* transfection kit (Mirus Bio LLC, Madison, WI). At 6 hours post transfection, the media was removed, and cells were washed once with complete media. 1 mL of complete growth media was added per well, and the cells were incubated at 37 °C until the desired time point. At 6, 12, 24, and 48 hours post transfection, cells were scraped into the supernatant, and the samples were collected into individual cryotubes and stored at -80 °C until all samples were collected. Samples were subjected to 3 freeze-thaw cycles and cleared by centrifugation at 2500 rpm for 5 minutes at 4 °C. Cleared lysates were titrated on H1-HeLa cells, and the TCID₅₀ per mL of each sample was determined using the Spearman-Kärber equation. Virus production from the Kelly *et al.* construct, CVA21-3'miRT, was severely impaired. The rate of rescue from CVA21-TR(2x) RNA was slightly delayed compared to unmodified CVA21 RNA. All other miRT-CVA21 RNAs rescued virus at rates and levels similar to the unmodified RNA genome (Figure 2B). Similar results were obtained when the time course was conducted in the human melanoma cell line Mel624 (Imanis Life Sciences, Rochester, MN) (Figure 2C).

### Characterization of miRT-CVA21 viruses

In order to generate virus stocks, viral RNA were produced as described above. 2.5 µg RNA per well was transfected into H1-HeLa cells seeded in 6-well plates as described above. At 48-72 hours post transfection, the cells were scraped into the supernatant, and the samples were collected. The samples were subjected to 3 freeze-thaw cycles, cleared by centrifugation at 2500 rpm for 5 minutes at 4 °C, and filtered through a 0.22 µm syringe filter. H1-HeLa cells in a T75 flask were infected with the cleared lysates at 37 °C in serum-free media. Two hours post infection, the media and unincorporated virus were removed, and the cells were replenished with complete growth media. Once CPE was observed, the samples were processed in the same manner. The cleared lysate virus stocks were aliquoted and stored at -80 °C. Viruses were titrated on H1-HeLa cells. 1 x 10⁴ cells per well were plated in 96-well tissue-culture plates, 24 hours prior to infection. Ten-fold serial dilutions (1 x 10⁻² to 1 x 10⁻¹⁰) of the virus were made in serum-free media, and 100 µL of each dilution was added to each of 8 duplicate wells. The cells were infected for 2 hours at 37 °C, and then the media was removed and replaced with 100 µL complete growth media. The cells were incubated at 37 °C for 72 hours, and then the wells were visually inspected for CPE. The TCID₅₀ per mL of each sample was determined using the Spearman-Kärber equation.

One-step growth curves were performed to compare the replication kinetics of the miRT-CVA21 to the unmodified virus. H1-HeLa cells were infected with unmodified or miRT-CVA21 at a multiplicity of infection of 3.0 in serum-free media. Two hours post infection, the cells were washed, and complete growth media was added. Samples were collected at specific times post transfection (2, 4, 6, 8, 12, 24, and 48 hours) and stored at -80° C. Following the completion of all timepoints, samples were frozen and thawed 3 times, and cellular debris was cleared from the lysates by centrifugation at 2500 rpm for 5 minutes at 4 °C. The cleared lysates were then titrated, and virus titers were determined using the Spearman-Kärber equation. All miRT-CVA21 replicated with kinetics similar to the unmodified virus.

The efficiency and specificity of microRNA-targeting were analyzed by measuring cell viability and viral replication in H1-HeLa cells transfected with complementary or noncomplementary synthetic miRNA mimics (Dharmacon, Lafayette, CO). Mimics were reverse transfected into H1-HeLa cells using the *TransIT-mRNA* transfection kit according to the manufacturer's protocol at a concentration of 100 nM each. Briefly, transfection complexes were assembled in a 96-well plate and incubated at room temperature for 5 minutes. H1-HeLa cells in T75 flasks were trypsinized, counted, and resuspended in complete growth media at a concentration of 1 x 10⁴ cells per 90 µL. 90 µL of cells was added per well, and the cells/transfection mixtures were incubated at 37 °C for 12 hours. The cells were infected at an MOI of 1 with each miRT-CVA21 for 2 hours at 37 °C in serum-free media. Following infection, the media and unincorporated virus was removed and replaced with 100 µL complete growth media, and the cells were incubated at 37 °C. 24 hours post infection, the supernatants were collected and titrated as described above. The cells were assayed for proliferation using a 3-(4,5-dimethylthiazolyl-2)-2,5-Diphenyltetrazolium bromide (MTT) kit (ATCC, Manassas, VA). CVA21-3'TR(2x) replication was not regulated by miR-133, miR-206, or a combination of the two mimics. Virus replication was minimally controlled by miR-133 for CVA21-686(2x) and CVA21-ΔV(2x). MicroRNA-206 was much more efficient at controlling virus replication resulting in increased cell viability and decreased virus titers for CVA21-ΔV(1x), CVA21-ΔV(2x), CVA21-686(2x), and CVA21-3'TR(1x). Virus tropism was similarly regulated in H1-HeLa cells transfected with both miR-133 and miR-206. No difference in cell viability and virus titer was observed in H1-HeLa cells transfected with the miR-142 control mimic or non-transfected cells. Of note, viruses with 5' UTR localized REs were more readily controlled than CVA21-3'TR(1x). Based on these results, the analyses did not continue with the CVA21-3'TR(2x) construct.

### In Vitro Genetic Stability of Response Elements

The genetic stability of RE at variable locations was evaluated by force passaging miRT-CVA21 in TE671 muscle cells that express miR-133 and miR-206. TE-671 cells were cultured in 2% horse serum for 4 days, which induces the cells to differentiate into myotubes expressing higher levels of miR-133 and miR-206. Differentiated TE-671 cells (dTE-671) in 6-well tissue culture plates were infected at an MOI of 10 with CVA21-ΔV(1x), CVA21-ΔV(2x), CVA21-686(2x), CVA21-3'TR(1x), or CVA21-3'TR(2x) for 2 hours at 37 °C in serum-free media. After 2 hours, the media and unincorporated virus were removed, and the cells were washed with complete media. 1.5 mL of complete media was added per well, and the cells were incubated at 37 °C. At 24 hours post infection, the cells were scraped into the supernatant, and the samples were collected. All samples were subjected to 3 freeze-thaw cycles, and the lysates were clarified by centrifugation at 2500 rpm for 5 minutes at 4 °C and filtered through a 0.22 µm filter. Virus in clarified lysates was passaged serially in dTE-671 cells seven times, each time using 1 volume of clarified lysate to 2 volumes of fresh media. Viral RNA was isolated from the cleared lysates of each passage with a QIAamp viral RNA mini kit (Qiagen, CA) according to the manufacturer's instructions. cDNA was synthesized, and regions containing the REs were amplified. Amplicons were sequenced with nested primers. This assay was conducted in duplicate. Escape mutants were observed in CVA21-686(2x) samples as early as passage 2. All other miRT-CVA21 infections gave rise to escape mutants between passages 4 and 7.

### In Vivo Analysis of Oncolytic Activity

Based on the *in vitro* results, the CVA21-3'TR(2x) construct was not assessed *in vivo.* 4-5 week old female CB17 ICR-SCID mice were purchased from Envigo (Huntingdon, Cambridgeshire, UK). The mice were irradiated and 24 hours later implanted subcutaneously with 5e6 Mel624 cells in the right flank. When the tumors reached an average of 0.5 cm x 0.5 cm, the tumors were treated with 30 µg of RNA in 50 µL of saline intratumorally. Tumor volume, measured using a hand-held caliper, weights, and overall health were routinely monitored. Blood was collected from all mice on day 7 post RNA treatment. Mice were anesthetized through the inhalation of isoflurane, and blood was collected from the submandibular vein in a BD microtainer tube with a sera separator gel. Blood was allowed to coagulate for 30 minutes at room temperature, and then sera was separated by centrifugation at 8000 rpm for 5 minutes at 4 °C. Sera was stored at -80 °C. Virus in sera was titrated as described for virus stocks on H1-HeLa cells. Sera (bled via cardiac puncture) and skeletal muscle tissue were obtained from all mice at the time of euthanasia. Skeletal muscle sections were immediately flash frozen and stored at -80 °C or were fixed in 10% formalin. Total RNA was isolated from flash frozen tissue sections with an RNeasy Plus Universal mini kit (Qiagen, CA) according to the manufacturer's instructions. Viral RNA was isolated from sera using a QIAamp viral RNA mini kit (Qiagen, CA) according to the manufacturer's instructions. cDNA was synthesized, and regions containing the REs were amplified using the Titan One-Tube RT-PCR system (Sigma Aldrich, St. Louis, MO) according to the manufacturer's instructions. Amplicons were sequenced with nested primers.

Tumor volumes and weights of all mice throughout the duration of the experiment are shown in Figure 4A. Control treated mice and mice administered CVA21-3'miRT RNA all exhibited progressive tumor growth and were euthanized due to tumor volume exceeding 10% body weight or tumor ulceration. One mouse treated with CVA21-3'miRT was found in a moribund state and was immediately euthanized. Sequence analysis of viral genomes in skeletal muscle tissue from this mouse revealed wild-type reversions. Rapid tumor regression was observed in all mice treated with CVA21, CVA21-ΔV(1x), CVA21-ΔV(2x), CVA21-686(2x), or CVA21-3'TR(1x) RNA. Toxicity in the form of hind-limb paralysis (HLP), sudden death (FD), or excessive weight loss (WL) was observed in all mice treated with CVA21 RNA and a proportion of mice treated with CVA21-ΔV(1x), CVA21-686(2x), or CVA21-3'TR(1x) RNA at 60%, 60%, and 75%, respectively. No toxicity was observed in mice treated with CVA21-ΔV(2x). Toxicities observed and proportions per group are shown in Figure 4B. All mice treated with CVA21-ΔV(2x) appeared healthy and tumor-free at the end of study day 90. Viral genomes isolated from sera and skeletal muscle from all CVA21-ΔV(2x) treated mice maintained the RE without mutations. Overall survival for mice treated with CVA21-ΔV(2x) was 100%, significantly improving survival over control treated mice, *p* = 0.002 (Figure 4C).

As shown in Figure 4D, no infectious virus was recovered from sera isolated on day 7 post treatment from mice treated with CVA21-3'miRT RNA. In contrast, viral titers observed in sera from mice treated with CVA21-ΔV(1x), CVA21-ΔV(2x), CVA21-686(2x), or CVA21-3'TR(1x) RNA were at levels similar to those found in mice treated with CVA21 RNA.

### In Vivo Dose Escalation of CVA21-ΔV(2x) RNA

4-5 week old female CB17 ICR-SCID mice from Envigo (Huntingdon, Cambridgeshire, UK) were irradiated and 24 hours later implanted subcutaneously with 5e6 Mel624 cells in the right flank. When the tumors reached an average of 0.5 cm x 0.5 cm, the tumors were treated with 1-32 µg of CVA21-ΔV(2x) RNA in 50 µL of saline intratumorally. Tumor volume, measured using a hand-held caliper, weights, and overall health were routinely monitored. Blood was collected from all mice on day 9 post RNA treatment. Tumor size, weight, blood, sera isolation, tissue collection, and genome sequencing were all measured, obtained, processed and analyzed as described for the previous *in vivo* experiment.

As shown in Figure 5A, control treated mice exhibited progressive tumor growth, and all were euthanized due to tumor size or ulceration. 4 of 5 mice treated with 1 µg CVA21-ΔV(2x) RNA also exhibited progressive tumor growth, however, complete tumor regression was observed in one mouse. Complete tumor regression was observed in all other mice treated with CVA21-ΔV(2x) RNA at 4 to 32 µg. Hind-limb paralysis was observed in a single mouse treated with CVA21-ΔV(2x) RNA at 8 µg. Sequence analysis and histological analysis of skeletal muscle did not reveal any mutations in the response element sequence or signs of myositis. Another mouse treated with 32 µg of CVA21-ΔV(2x) RNA was found dead at day 78 post RNA treatment. The majority of mice treated with 4 to 32 µg of CVA21-ΔV(2x) RNA displayed high viral loads in sera on day 9 post RNA treatment, however, a few did not (Figure 5B). Time course analysis of viral loads in sera following RNA treatment can be used to establish when peak viral loads will be observed. Viremia was only observed in one mouse from the 1 µg group, and this mouse displayed complete tumor regression.

### Bilateral tumor destruction following CVA21-ΔV(2x) RNA therapy

4-5 week old female CB17 ICR-SCID mice are irradiated and 24 hours later are implanted subcutaneously with 5e6 Mel624 cells in the right flank and 5e6 Mel624 cells in the left flank. When tumors reach an average of 0.5 cm x 0.5 cm, the mice are treated. Each mouse is given a single injection of 2, 10, or 30 µg of CVA21-ΔV(2x) RNA in the right flank tumor. Tumor volume, which is measured using a hand-held caliper, weights, and overall health are routinely monitored. Blood is collected from two mice per group on days 2, 4, 6, 8 or 10 post treatment. Tumor size, weight, blood, sera isolation, tissue collection and genome sequencing is measured, is obtained, is processed and analyzed as described for the previous *in vivo* experiments.

### Potential infectious nucleic acid formulations for CVA21-ΔV(2x) therapy

Examples of infectious nucleic acid formulations include, but are not limited to, infectious cDNA clones or RNA transcripts encoding picornavirus genomes. Techniques used to synthesize viral RNA genomes from infectious cDNA can result in additional nucleotides (not part of the viral genome) on the 5' and/or 3' ends. Several positive and negative RNA viruses, including poliovirus, have been shown to require exact termini for efficient replication (Boyer et al., Virology, 198:415-426 (1994); Herold and Andino, J. Virol., 74(14):6394-6400 (2000)). Although these residues can be removed during replication generating the authentic viral genomes, their initial presence can have detrimental effects on the specific infectivity of the therapeutic nucleic acid. CVA21-ΔV(2x) infectious nucleic acid therapy may be improved by employing mechanisms to rapidly generate authentic viral genomes. One such mechanism is to encode ribozyme sequences at the 5' and/or 3' termini. Ribozymes are RNA molecules (structures) with catalytic properties. Certain ribozymes are capable of cleaving RNA molecules in *cis* or in *trans* at very specific positions. Encoding ribozymes at the 5' and/or 3' end of the viral genome in infectious nucleic acid formulations encoding CVA21-ΔV(2x) may improve the therapeutic efficacy even further.

Three different constructs are made to confirm this. The first includes a ribozyme immediately upstream of the CVA21-ΔV(2x) genome (Rz- CVA21-ΔV(2x)). This ribozyme is modified to allow cleavage in *cis* at the exact 5' termini of the CVA21-ΔV(2x) genome. The second construct includes a different ribozyme immediately downstream of the CVA21-ΔV(2x) genome (CVA21-ΔV(2x)-Rz) such that it cleaves the RNA in *cis* directly downstream of the encoded poly A tail of CVA21-ΔV(2x). The third construct includes the CVA21-ΔV(2x) genome flanked by both of these ribozymes (Rz-CVA21-ΔV(2x)-Rz). RNA genomes are synthesized, and their specific infectivity, targeting efficacy/specificity, genetic stability, and therapeutic efficacy/safety are characterized as described above for the unmodified CVA21-ΔV(2x). A mechanism to ensure authentic or near authentic 3' termini is to include sequences encoding a restriction site that can be cleaved by a restriction endonuclease directly adjacent or within 5 nucleotide residues following the encoded 3' end (e.g. poly A tail). An NheI restriction enzyme site was encoding within the DNA encoding the viral genome CVA21-ΔV(2x) directly adjacent to the encoded poly A tail. The DNA was linearized with the NheI restriction endonuclease such that the in-vitro derived RNA transcripts encoding the CVA21-ΔV(2x) contained 5 non-viral nucleotides following the poly A tail (CVA21-ΔV(2x)3'NheI). Another construct was made with the NheI site at the 3' end of the CVA21-ΔV(2x) genome in conjunction with a ribozyme immediately upstream of the CVA21-ΔV(2x) genome (Rz- CVA21-ΔV(2x)-3'NheI). RNA genomes are synthesized, and their specific infectivity, targeting efficacy/specificity, genetic stability, and therapeutic efficacy/safety are characterized as described above for the unmodified CVA21-ΔV(2x).

### Therapeutic efficacy in a variety of tumor types

CVA21-ΔV(2x) infectious nucleic acid therapy can be used to treat a variety of cancer types including, but not limited to, melanoma, myeloma, prostate cancer, breast cancer, lung cancer (e.g., non-small cell lung cancer), and pancreatic cancer. 1 x 10⁴ cells per well of representative tumor cell lines for these cancer types were plated in 96-well tissue culture dishes. The cells were infected at an increasing MOI between 0.001 and 1 with CVA21 or CVA21-ΔV2 for 2 hours at 37 °C in serum-free media. Following infection, the media and unincorporated virus was removed and replaced with 100 µL complete growth media, and the cells were incubated at 37 °C. 72 hours post infection, the cells were assayed for proliferation using a 3-(4,5-dimethylthiazolyl-2)-2,5-Diphenyltetrazolium bromide (MTT) kit (ATCC, Manassas, VA). All cell lines tested were as susceptible to CVA21-ΔV2 as they were to CVA21 (Figure 6).

This tumor cell panel is analyzed for susceptibility to infectious nucleic acid formulations encoding CVA21-ΔV2. 6 x 10⁴ cells per well are plated in 24-well tissue culture dishes. RNA transcripts encoding CVA21, CVA21-ΔV2, Rz-CVA21-ΔV2, CVA21-ΔV2-Rz, or Rz-CVA21-ΔV2-Rz are transfected into the cells. Each well is transfected with 0.5 µg of purified T7 RNA using *TransIT-mRNA* transfection kit (Mirus Bio LLC, Madison, WI). No template transfection controls are used to account for changes in cell viability associated with the transfection protocol. At 24, 48, and 72 hours post transfection, cells are assayed for proliferation using a 3-(4,5-dimethylthiazolyl-2)-2,5-Diphenyltetrazolium bromide (MTT) kit (ATCC, Manassas, VA).

### Example 2 - Infectivity and stability of nucleic acid that can be used to treat cancer Insertion of a 2x RE in domain Y reduces specific infectivity and eliminates therapeutic efficacy of infectious RNA encoding the CVA21-3'miRTgenome.

Recovery of infectious virus following transfection of 1 µg of in vitro-derived infectious RNA encoding CVA21-3'miRT was severely delayed in both H1-HeLa and Mel624 cells compared to RNA encoding unmodified CVA21 (Figure 2). 4-5 week old female CB17 ICR-SCID mice were purchased from Envigo (Huntingdon, Cambridgeshire, UK). The mice were irradiated and 24 hours later implanted subcutaneously with 5e6 Mel624 cells in the right flank. When the tumors reached an average of 0.5 cm x 0.5 cm, the tumors were treated with 30 µg of RNA encoding either unmodified CVA21 or CVA21-3'miRT in 50 µL of saline intratumorally. Tumor volume, measured using a hand-held caliper, weights, and overall health were routinely monitored. Blood was collected from all mice on day 7 post RNA treatment. Mice were anesthetized through the inhalation of isoflurane, and blood was collected from the submandibular vein in a BD microtainer tube with a sera separator gel. Blood was allowed to coagulate for 30 minutes at room temperature, and then sera was separated by centrifugation at 8000 rpm for 5 minutes at 4 °C. Sera was stored at -80 °C. Virus in sera was titrated as described for virus stocks on H1-HeLa cells. As shown in Figures 4 A and D, RNA encoding CVA21-3'miRT did not exhibit any oncolytic activity or induce viremia. RNA secondary structural prediction demonstrates the disruption of the Y domain of the oriR and reduced probability of the pseudoknot formation that likely contributes to the reduced specific infectivity of the in vitro-derived RNA and lack of therapeutic efficacy (Figure 13).

### Scanning region replacement enhances microRNA response element stability.

At the time of euthanasia, viral genomes were isolated from the sera and skeletal muscle of mice treated with CVA21-ΔV(2x) and mice with clinical signs of toxicity. Viral or total RNA was isolated from the samples, respectively, and cDNA was synthesized. Regions containing the microRNA response elements were amplified and the amplicons sequenced. Reversion mutants were detected in the skeletal muscle of one mouse treated with CVA21-ΔV(1x) that developed hind-limb paralysis and in all three mice treated with CVA21-686(2x) that developed hind-limb paralysis. In contrast, no reversion or escape mutants were detected in any of the mice treated with CVA21-ΔV(2x). Of note, reversion mutants were also detected in both of the evaluable mice treated with CVA21-TR(1x). In an effort to determine the stability of the microRNA response elements *in vitro,* serial passage was performed of each microRNA-detargeted CVA21 in differentiated TE671 (dTE-671) muscle cells that express miR-133 and miR-206. dTE671 cells were initially infected at an MOI of 10. At twenty-four hours post infection, the samples were collected and fresh dTE671 cells infected with 50% of the clarified lysates. Viral RNA was isolated from cleared lysates of seven serial passages, cDNA synthesized and regions containing the response elements amplified for sequencing. In both experiments, reversion mutants were detected in CVA21-686(2x) samples 2-3 passages prior to detection in CVA21-ΔV(2x) and CVA21-ΔV(1x) samples. This data indicates that elongation of the scanning region with direct microRNA response element insertion decreases the genetic stability, an effect that is more pronounced *in vivo.*

### Replacement of the ribosomal scanning region with microRNA response element minimizes potential for structural alterations.

In contrast to the complex structural environment of the 3' NCR, the 5' NCR of CVA21 is predicted to contain a disordered scanning region directly downstream of the IRES. The role of this domain in CVA21 replication is unknown. Although this region has been shown to be dispensable for poliovirus replication *in vitro* and *in vivo,* it has been indicated in binding an IRES *trans-*acting factor that enhances enterovirus 71 translation. RNA secondary structural analysis predicted that the CVA21-ΔV(2x) configuration resulted in maintenance of domain VI within the IRES and therefore should not significantly impact ribosomal loading and translation (Figure 13). No pseudoknot formations are impacted by insertion within the 5' NCR as is the case with insertion anywhere within the 3' NCR. As shown in Figure 13D-F, although the TR(2x) construct separates the microRNA response element from the oriR loops (domains Y and X), the predicted pseudoknot interaction between domain Y and domain X is still lost as depicted by the interloop connecting lines.

### Potential infectious nucleic acid formulations for CVA21-ΔV(2x) therapy

CVA21-ΔV(2x) infectious nucleic acid therapy may be improved by employing mechanisms to rapidly generate authentic viral genomes. One such mechanism is to encode ribozyme sequences at the 5' and 3' termini. Ribozymes are RNA molecules (structures) with catalytic properties. Certain ribozymes are capable of cleaving RNA molecules in *cis* or in *trans* at very specific positions. Encoding ribozymes at the 5' and/or 3' end of the viral genome in infectious nucleic acid formulations encoding CVA21-ΔV(2x) may improve the therapeutic efficacy even further.

Five different constructs were made to test this hypothesis. The first included a ribozyme immediately upstream of the CVA21-ΔV(2x) genome (Rz-CVA21-ΔV(2x)). This ribozyme was modified to allow cleavage in *cis* at the exact 5' termini of the CVA21-ΔV(2x) genome. The second construct included a different ribozyme immediately downstream of the CVA21-ΔV(2x) genome (CVA21-ΔV(2x)-Rz) such that it will cleave the RNA in *cis* directly downstream of the encoded poly A tail of CVA21-ΔV(2x). The third construct included an NheI restriction enzyme cut site directly adjacent to the poly A tail of the viral genome encoded in the plasmid DNA. The NheI cut site was used to generate linear transcripts such that only a few residues will follow the poly A tail. The fourth construct included a ribozyme at the 5' termini of the CVA21-ΔV(2x) genome and the NheI cut site following the poly A tail (Rz- CVA21-ΔV(2x)-3'NheI). The fifth construct included the CVA21-ΔV(2x) genome flanked by both of these ribozymes (Rz-CVA21-ΔV(2x)-3'Rz). RNA genomes were synthesized and their specific infectivity, targeting efficacy/specificity, genetic stability, and therapeutic efficacy/safety characterized as described above for the unmodified CVA21-ΔV(2x). As shown in Figure 14, both constructs with dual authentic termini or near-authentic (i.e., Rz-CVA21-ΔV(2x)-3'Rz and Rz-CVA21-ΔV(2x)-3'NheI, respectively) had increased specific infectivity and cytopathic effects observed sooner following transfection of H1-HeLa cells compared to the unmodified CVA21-ΔV(2x) RNA. Both of these constructs exhibit oncolytic activity against subcutaneous Mel624 xenograft tumors in CB-17 SCID mice (Figure 15A). Both constructs were also able to generate spreading oncolytic infections as exhibited by the development of viremia in the treated mice on days 2 and 7 post therapy (Figure 15B). Rz-CVA21-ΔV(2x)-3'NheI was more efficient than CVA21-ΔV(2x) and Rz-CVA21-ΔV(2x)-3'Rz and the development of viremia in mice treated with Rz-CVA21-ΔV(2x)-3'NheI was more consistent. Different combinations of ribozyme-encoding sequences and restriction enzyme cut sites (with exogenously added restriction enzymes designed to cut at those cut sites) may be used to modulate the specific infectivity and therapeutic potential of the infectious RNA formulation. This may include but is not limited to the immunogenic potential of the construct in various tumor microenvironments and delivery routes.

### Initial seeding of heterogenous tumor cells is independent of virus receptor expression, but spread and safety is still dependent on the expression of hICAM-1 and/or decay-accelerating factor.

Murine melanoma B16-F10 cells are not susceptible to CVA21 because they do not express the virus receptors. A B16-F10 cell line stably expressing hICAM-1 was generated via lentiviral transduction, antibiotic selection, single-cell sorting and expansion of a clonal population. Virus recovery from this cell line B16-F10-hICAM1 24 hours post infection with CVA21-ΔV(2x) at an MOI of 1 was significantly enhanced compared to the parental cell line (Figure 16). This effect can also be applied to other cell lines that do not express the receptors for virus entry to expand the repertoire of *in vivo* models. Infectious nucleic acid can be used to expand initial seeding of tumor cells to include cells that do not express the receptor for the virus while maintaining the specificity of spread following virus production. Cell type specificity can be further modulated by incorporation of different microRNA target sequences.

### In vitro transcription reactions can be scaled to generate clinical preps of CVA21-ΔV(2x) infectious RNA

Preclinical studies utilized an *in vitro* RNA transcription kit to generate RNA transcripts. Each 10 microliter reaction would yield ~100 micrograms of RNA that was used in the *in vivo* studies. To demonstrate the feasibility of scaling a production method without commercially available kits, a similar reaction was set up and scaled to 1 mL (100x), and the resulting transcripts were purified using lithium chloride precipitation. This scaled reaction resulted in >7 milligrams of RNA with integrity similar to 10 microliter reactions as observed by RNA gel electrophoresis (Figure 17).

### Modifications to in vitro-derived infectious nucleic acid can be used to enhance stability in different environments.

Various mechanisms can be used to enhance the stability of RNAs in blood and translatability following internalization within a cell. These techniques include, but are not limited to, using modified bases (e.g., N-methylpseudouridine) in the *in vitro* transcription to generate RNAs that closely resemble RNAs normally present within the hosts being treated, capping mechanisms, and polyadenylation of transcripts. All of these mechanisms and others to enhance the stability, delivery, uptake, and expression of nucleic acid based therapeutics can be applied to infectious nucleic acid therapy. These mechanisms can also be used to modulate the immunogenicity of the infectious nucleic acid on a per patient basis. Additionally, the delivery of infectious nucleic acid can be enhanced by complexing the nucleic acid with a variety of different carrier molecules, including, but not limited to, lipid-based, polymer-based, nanoparticle-based, and other biosynthetic molecules.

### Example 3: Reduced cost and simplification of manufacturing

Manufacturing protocols vary for each oncolytic virus, however, the general outline of procedures necessary to produce and purify large quantities of the virus with sufficient titers of infectious particles is similar. A GMP master cell bank and master seed virus are generated and qualified. These cells are seeded and expanded using optimized medium formulations until a sufficient number of cells are established. These cells are often seeded into large bioreactors for further expansion and infection. The virus is harvested, generally requiring lysis and/or clarification of cellular debris followed by enzymatic digestion of residual nucleic acids and purification of the lysate. Downstream purification processes can include various ultrafiltration and difiltration steps and chromatography (e.g. ion exchange and gel permeation) based purification. This is followed by another filtration step prior to vialing and storing. Production of RNA-based therapeutics requires fewer steps and can be scaled to produce higher yields in lower volumes (i.e., smaller bioreactors). The steps involved in producing infectious RNA are fewer and simpler. A master bank of linearized plasmid DNA encoding the viral genome is made. This is used for *in vitro* transcription reactions (generally ~1 L per stock) to produce the infectious RNA. DNase digestion is used to remove residual plasmid DNA, and the infectious RNA is then purified via precipitation or column filtration. HPLC or FPLC purification can be used to remove additional contaminants to ensure purity. In contrast to current mRNA-based therapeutics, CVA21-ΔV(2x) transcripts do not require capping or tailing reducing the costs/steps of synthesis and purification using *in vitro* transcription GMP protocols. Oncolytic CVA21 viruses can be used clinically in combination with immunotherapy.

### Example 4: Potential for enhancing virus monotherapy

Formulating CVA21 as infectious nucleic acid has the potential to safely enhance its monotherapeutic potency. Nucleic acid is less immunogenic than virus particles providing a mechanism to avoid neutralizing antibodies during repeat dosing. Infectious nucleic acid delivery can infect cells even in the presence of neutralizing antibodies boosting the oncolytic phase of therapy during repeat injections that may enhance the overall efficacy of treatment. Furthermore, infectious nucleic acid is not restricted to tumor cells expressing the virus receptor (e.g., human intracellular adhesion molecule I). The initial seeding of tumor cells will include those cells normally refractory to virus infection, overcoming the barrier of tumor heterogeneity. This strategy can be applied to other oncolytic picornaviruses.

### Example 5: Improved safety expands patient eligibility

The tolerability of CVA21 has been demonstrated in phase I and II clinical trials in patients with several advanced malignancies. However, studies to date have been limited to patients with functional immune systems. Although rare in humans, CVA21 can cause myositis with immunodeficient hosts being particularly vulnerable. Thus, development of a CVA21 that is unable to replicate in muscle cells will allow expansion of clinical analyses to patients with compromised immune systems. CVA21-ΔV(2x) has a microRNA response element that includes sequences recognized by microRNAs enriched within muscle tissues. This element reduces viral replication in cells expressing the cognate microRNAs and ameliorates toxicity observed in immunodeficient mice bearing subcutaneous tumors treated with CVA21. MicroRNA-detargeted viruses including CVA21-ΔV(2x) can be used to improve safety of oncolytic therapies in immunocompromised patients.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EMBODIMENTS

Although the present invention is defined in the claims, it should be understood that the present invention can also (alternatively) be defined in accordance with the following embodiments:
1. A nucleic acid construct comprising an infectious nucleic acid comprising a picornavirus genome comprising one or more heterologous sequence elements of 20 or more bases, wherein the specific infectivity of said construct is sufficient to initiate a spreading picornavirus infection when administered to a living mammal, wherein said specific infectivity of said construct is of similar magnitude to the specific infectivity of a comparable construct lacking said one or more heterologous sequence elements.
2. The construct of embodiment 1, wherein said mammal is a human.
3. The construct of any one of embodiments 1-2, wherein said construct is formulated as plasmid DNA.
4. The construct of any one of embodiments 1-3, wherein said construct is formulated as an RNA molecule.
5. The construct of any one of embodiments 1-4, wherein at least one of said one or more heterologous sequence elements is a microRNA response element.
6. The construct of embodiment 5, wherein a microRNA target element of said microRNA response element comprises at least a region of complementarity to a microRNA present in non-cancer cells.
7. The construct of any one of embodiments 1-6, wherein at least one of said one or more heterologous sequence elements is a tissue-specific microRNA response element.
8. The construct of any one of embodiments 1-7, wherein at least one of said one or more heterologous sequence elements is a muscle-specific, brain-specific, or heart-specific microRNA response element.
9. The construct of any one of embodiments 1-8, wherein at least one of said one or more heterologous sequence elements is inserted into the 5' non-coding region of said picornavirus genome as a substitution for nucleotides within a scanning region.
10. The construct of any one of embodiments 1-9, wherein said picornavirus genome comprises a type I internal ribosome entry site.
11. The construct of any one of embodiments 1-10, wherein said picornavirus genome is a coxsackievirus, poliovirus, echovirus, rhinovirus, or enterovirus genome.
12. The construct of any one of embodiments 1-11, wherein said picornavirus genome is a coxsackievirus A21 genome.
13. The construct of any one of embodiments 1-12, wherein said picornavirus genome comprises a microRNA target element for miR-133.
14. The construct of any one of embodiments 1-13, wherein said picornavirus genome comprises more than one microRNA target element for miR-133.
15. The construct of any one of embodiments 1-14, wherein said picornavirus genome comprises a microRNA target element for miR-206.
16. The construct of any one of embodiments 1-15, wherein said picornavirus genome comprises more than one microRNA target element for miR-206.
17. The construct of any one of embodiments 1-16, wherein said picornavirus genome comprises more than one microRNA target element for miR-133 and more than one microRNA target element for miR-206.
18. The construct of any one of embodiments 1-17, wherein said picornavirus genome is a coxsackievirus A21 genome, and wherein said picornavirus genome lacks at least 20 nucleotides from position 631 to position 698 of a wild-type coxsackievirus A21 genome.
19. The construct of any one of embodiments 1-18, wherein said picornavirus genome is a coxsackievirus A21 genome, and wherein said picornavirus genome lacks nucleotides 631 to 698 of a wild-type coxsackievirus A21 genome.
20. The construct of any one of embodiments 1-19, wherein said nucleic acid construct is DNA, and said nucleic acid construct encodes a ribozyme.
21. The construct of embodiment 20, wherein said ribozyme is designed to cleave a 5' portion of RNA from an RNA molecule transcribed from said nucleic acid construct, thereby creating a ribozyme-cleaved RNA.
22. The construct of embodiment 20, wherein said ribozyme-cleaved RNA comprises a 5' end with no ribonucleotides that are not present in a picornavirus genome.
23. The construct of any one of embodiments 1-19, wherein said nucleic acid construct is RNA, and said nucleic acid construct comprises a ribozyme.
24. The construct of embodiment 23, wherein said ribozyme is designed to cleave a 5' portion of RNA from said nucleic acid construct, thereby creating a ribozyme-cleaved RNA.
25. The construct of embodiment 24, wherein said ribozyme-cleaved RNA comprises a 5' end with no ribonucleotides that are not present in a picornavirus genome.
26. The construct of any one of embodiments 1-19, wherein said nucleic acid construct is DNA, and said nucleic acid construct comprises a restriction endonuclease cut site.
27. The construct of embodiment 26, wherein said restriction endonuclease cut site is designed to allow for cleavage, via a restriction endonuclease, of a 3' portion of said nucleic acid construct, thereby creating a restriction endonuclease-cleaved nucleic acid construct.
28. The construct of embodiment 27, wherein said restriction endonuclease-cleaved nucleic acid construct encodes a virus comprising a 3' end with less than 10 ribonucleotides that are not present in a picornavirus genome.
29. The construct of embodiment 27, wherein said restriction endonuclease-cleaved nucleic acid construct encodes a virus comprising a 3' end with no ribonucleotides that are not present in a picornavirus genome.
30. The construct of any one of embodiments 1-19, wherein said nucleic acid construct is DNA, said nucleic acid construct encodes a ribozyme, and said nucleic acid construct comprises a restriction endonuclease cut site.
31. The construct of embodiment 30, wherein said ribozyme is designed to cleave a 5' portion of RNA from an RNA molecule transcribed from said nucleic acid construct, thereby creating a ribozyme-cleaved RNA.
32. The construct of embodiment 30, wherein said ribozyme-cleaved RNA comprises a 5' end with no ribonucleotides that are not present in a picornavirus genome.
33. The construct of embodiment 30, wherein said restriction endonuclease cut site is designed to allow for cleavage, via a restriction endonuclease, of a 3' portion of said nucleic acid construct, thereby creating a restriction endonuclease-cleaved nucleic acid construct.
34. The construct of embodiment 30, wherein said restriction endonuclease-cleaved nucleic acid construct encodes a virus comprising a 3' end with less than 10 ribonucleotides that are not present in a picornavirus genome.
35. The construct of embodiment 30, wherein said restriction endonuclease-cleaved nucleic acid construct encodes a virus comprising a 3' end with no ribonucleotides that are not present in a picornavirus genome.
36. A method of reducing the number of cancer cells within a living mammal, wherein said method comprises administering a construct set forth in any one of embodiments 1-35 to the mammal.
37. The method of embodiment 36, wherein said mammal is a human.
38. The method of any one of embodiments 36-37, wherein said cancer cells are melanoma, pancreatic, prostate, bladder, non-small cell lung, myeloma, or breast cancer cells.
39. The method of any one of embodiments 36-38, wherein said administering step results in a reduced number of non-cancerous cells present within said mammal undergoing cell lysis following said administering step as compared to the number of non-cancerous cells that undergo lysis when said comparable construct is administered to a comparable mammal.
40. The method of any one of embodiments 36-39, wherein said administering step results in a similar number or an increased number of cancerous cells present within said living mammal undergoing cell lysis following said administering step as compared to the number of cancerous cells that undergo lysis when said comparable construct is administered to a comparable mammal.
41. A method of reducing the number of cancer cells within a living mammal, wherein said method comprises administering a construct set forth in any one of embodiments 1-35 to the mammal, wherein said cancer cells undergo lysis as a result of unencumbered synthesis of corresponding picornaviruses from said construct, thereby reducing the number of cancer cells within said living mammal.
42. The method of embodiment 41, wherein said mammal is a human.
43. The method of any one of embodiments 41-42, wherein said cancer cells are melanoma, myeloma, or breast cancer cells.
44. The method of any one of embodiments 41-43, wherein said administering step results in a reduced number of non-cancerous cells present within said mammal undergoing cell lysis following said administering step as compared to the number of non-cancerous cells that undergo lysis when said comparable construct is administered to a comparable mammal.
45. The method of any one of embodiments 41-44, wherein said administering step results in a similar number or an increased number of cancerous cells present within said living mammal undergoing cell lysis following said administering step as compared to the number of cancerous cells that undergo lysis when said comparable construct is administered to a comparable mammal.
46. The method of any one of embodiments 41-45, said method further comprising administering an anti-cancer agent to said mammal.
47. The method of embodiment 46, wherein said anti-cancer agent is a checkpoint inhibitor.
48. The method of embodiment 47, wherein said checkpoint inhibitor is selected from the group consisting of a CTLA-4 inhibitor, a PD-1 inhibitor, and a PD-L1 inhibitor.
49. An isolated infectious nucleic acid encoding a coxsackievirus, wherein said infectious nucleic acid lacks at least 20 nucleotides from position 631 to position 698 of a wild-type coxsackievirus A21 genome, and wherein said infectious nucleic acid comprises a microRNA target element for a muscle-specific microRNA that is located between a VI domain and an authentic translation start site of said infectious nucleic acid.
50. The isolated infectious nucleic acid of embodiment 49, wherein said infectious nucleic acid lacks said nucleotides from position 631 to position 698.
51. The isolated infectious nucleic acid of any one of embodiments 49-50, wherein said muscle-specific microRNA is miR-133 or miR-206.
52. The isolated infectious nucleic acid of any one of embodiments 49-51, wherein said infectious nucleic acid comprises said microRNA target element between a first position and a second position, wherein said first position corresponds to position 631 of said wild-type coxsackievirus A21 genome, and wherein said second position corresponds to position 699 of said wild-type coxsackievirus A21 genome.
53. The isolated infectious nucleic acid of any one of embodiments 49-52, wherein said infectious nucleic acid is DNA, and said infectious nucleic acid encodes a ribozyme.
54. The isolated infectious nucleic acid of embodiment 53, wherein said ribozyme is designed to cleave a 5' portion of RNA from an RNA molecule transcribed from said infectious nucleic acid, thereby creating a ribozyme-cleaved RNA.
55. The isolated infectious nucleic acid of embodiment 54, wherein said ribozyme-cleaved RNA encodes a coxsackievirus comprising a 5' end with no ribonucleotides that are not present in a picornavirus genome.
56. The isolated infectious nucleic acid of any one of embodiments 49-52, wherein said isolated infectious nucleic acid RNA, and said isolate infectious nucleic acid comprises a ribozyme.
57. The isolated infectious nucleic acid of embodiment 56, wherein said ribozyme is designed to cleave a 5' portion of RNA from said infectious nucleic acid, thereby creating a ribozyme-cleaved RNA.
58. The isolated infectious nucleic acid of embodiment 56, wherein said ribozyme-cleaved RNA encodes a coxsackievirus comprising a 5' end with no ribonucleotides that are not present in a picornavirus genome.
59. The isolated infectious nucleic acid of any one of embodiments 49-52, wherein said infectious nucleic acid is DNA, and said infectious nucleic acid comprises a restriction endonuclease cut site.
60. The isolated infectious nucleic acid of embodiment 59, wherein said restriction endonuclease cut site is designed to allow for cleavage, via a restriction endonuclease, of a 3' portion of said infectious nucleic acid, thereby creating a restriction endonuclease-cleaved infectious nucleic acid.
61. The isolated infectious nucleic acid of embodiment 60, wherein said restriction endonuclease-cleaved infectious nucleic acid encodes a coxsackievirus comprising a 3' end with less than 10 ribonucleotides that are not present in a picornavirus genome.
62. The isolated infectious nucleic acid of embodiment 60, wherein said restriction endonuclease-cleaved infectious nucleic acid encodes a coxsackievirus comprising a 3' end with no ribonucleotides that are not present in a picornavirus genome.
63. The isolated infectious nucleic acid of any one of embodiments 49-52, wherein said infectious nucleic acid is RNA, said infectious nucleic acid encodes a ribozyme, and said infectious nucleic acid comprises a restriction endonuclease cut site.
64. The isolated infectious nucleic acid of embodiment 63, wherein said ribozyme is designed to cleave a 5' portion of RNA from an RNA molecule transcribed from said infectious nucleic acid, thereby creating a ribozyme-cleaved RNA.
65. The isolated infectious nucleic acid of embodiment 63, wherein said ribozyme-cleaved RNA comprises a 5' end with no ribonucleotides that are not present in a picornavirus genome.
66. The isolated infectious nucleic acid of embodiment 63, wherein said restriction endonuclease cut site is designed to allow for cleavage, via a restriction endonuclease, of a 3' portion of RNA from said infectious nucleic acid, thereby creating a restriction endonuclease-cleaved infectious nucleic acid.
67. The isolated infectious nucleic acid of embodiment 66, wherein said restriction endonuclease-cleaved infectious nucleic acid encodes a coxsackievirus comprising a 3' end with less than 10 ribonucleotides that are not present in a picornavirus genome.
68. The isolated infectious nucleic acid of embodiment 66, wherein said restriction endonuclease-cleaved infectious nucleic acid encodes a coxsackievirus comprising a 3' end with no ribonucleotides that are not present in a picornavirus genome.
69. A method for treating cancer present in a mammal, wherein said method comprises administering, to said mammal, an effective amount of infectious nucleic acid encoding a coxsackievirus, wherein said infectious nucleic acid lacks at least 20 nucleotides from position 631 to position 698 of a wild-type coxsackievirus A21 genome, and wherein said infectious nucleic acid comprises a microRNA target element for a muscle-specific microRNA that is located between a VI domain and an authentic translation start site of said infectious nucleic acid.
70. The method of embodiment 69, wherein said mammal is a human.
71. The method of any one of embodiments 69-70, wherein said cancer is melanoma, myeloma, or breast cancer.
72. The method of any one of embodiments 69-71, wherein said infectious nucleic acid lacks said nucleotides from position 631 to position 698.
73. The method of any one of embodiments 69-72, wherein said muscle-specific microRNA is miR-133 or miR-206.
74. The method of any one of embodiments 69-73, wherein said infectious nucleic acid comprises said microRNA target element between a first position and a second position, wherein said first position corresponds to position 631 of said wild-type coxsackievirus A21 genome, and wherein said second position corresponds to position 699 of said wild-type coxsackievirus A21 genome.
75. The method of any one of embodiments 69-74, said method further comprising administering an anti-cancer agent to said mammal.
76. The method of embodiment 75, wherein said anti-cancer agent is a checkpoint inhibitor.
77. The method of embodiment 76, wherein said checkpoint inhibitor is selected from the group consisting of a CTLA-4 inhibitor, a PD-1 inhibitor, and a PD-L1 inhibitor.
78. A method for making infectious RNA comprising a picornavirus genome comprising one or more heterologous sequence elements of 20 or more bases, wherein the specific infectivity of said infectious RNA is sufficient to initiate a spreading picornavirus infection when administered to a living mammal, wherein said specific infectivity of said infectious RNA is of similar magnitude to the specific infectivity of a comparable infectious RNA lacking said one or more heterologous sequence elements, wherein said method comprises:
   (a) providing an DNA construct encoding said infectious RNA, wherein said DNA construct encodes a ribozyme, and wherein said nucleic acid construct comprises a restriction endonuclease cut site, and
   (b) contacting said DNA construct with a restriction endonuclease under conditions wherein at least a portion of said DNA construct is removed, thereby producing a restriction endonuclease-cleaved DNA construct,
   wherein said restriction endonuclease-cleaved DNA construct encodes an infectious RNA having non-picornavirus RNA located at a 5' end, and wherein said ribozyme cleaves said non-picornavirus RNA located at the 5' end to generate said infectious RNA.
79. The method of embodiment 78, wherein said infectious RNA encoded by said restriction endonuclease-cleaved DNA construct comprises a 3' end with less than 10 ribonucleotides that are not present in a picornavirus genome.
80. The method of embodiment 78, wherein said infectious RNA encoded by said restriction endonuclease-cleaved DNA construct comprises a 3' end with no ribonucleotides that are not present in a picornavirus genome.
81. The method of embodiment 78, wherein said infectious RNA comprises a 5' end with no ribonucleotides that are not present in a picornavirus genome.
82. An immunocompetent model that can be infected by infectious nucleic acid encoding a virus, wherein said immunocompetent model comprises a mouse cell expressing a human intracellular adhesion molecule 1 (ICAM-1).
83. The immunocompetent model of embodiment 82, wherein said mouse cell is a murine melanoma B16-F10 cell.

## Claims

1. An isolated infectious nucleic acid encoding a coxsackievirus, wherein said infectious nucleic acid lacks at least 20 nucleotides from position 631 to position 698 of a wild-type coxsackievirus A21 genome, and wherein said infectious nucleic acid comprises a microRNA target element for a muscle-specific microRNA that is located between a VI domain and an authentic translation start site of said infectious nucleic acid.

2. The isolated infectious nucleic acid of claim 1, wherein said infectious nucleic acid lacks said nucleotides from position 631 to position 698; and/or wherein said muscle-specific microRNA is miR-133 or miR-206; and/or wherein said infectious nucleic acid comprises said microRNA target element between a first position and a second position, wherein said first position corresponds to position 631 of said wild-type coxsackievirus A21 genome, and wherein said second position corresponds to position 699 of said wild-type coxsackievirus A21 genome.

3. The isolated infectious nucleic acid of any one of claims 1-2, wherein said infectious nucleic acid is DNA, and said infectious nucleic acid encodes a ribozyme, optionally wherein said ribozyme is designed to cleave a 5' portion of RNA from an RNA molecule transcribed from said infectious nucleic acid, thereby creating a ribozyme-cleaved RNA, and further optionally wherein said ribozyme-cleaved RNA encodes a coxsackievirus comprising a 5' end with no ribonucleotides that are not present in a picornavirus genome.

4. The isolated infectious nucleic acid of any one of claims 1-2, wherein said isolated infectious nucleic acid RNA, and said isolate infectious nucleic acid comprises a ribozyme, optionally wherein said ribozyme is designed to cleave a 5' portion of RNA from said infectious nucleic acid, thereby creating a ribozyme-cleaved RNA, or optionally wherein said ribozyme-cleaved RNA encodes a coxsackievirus comprising a 5' end with no ribonucleotides that are not present in a picornavirus genome.

5. The isolated infectious nucleic acid of any one of claims 1-2, wherein said infectious nucleic acid is DNA, and said infectious nucleic acid comprises a restriction endonuclease cut site.

6. The isolated infectious nucleic acid of claim 5, wherein said restriction endonuclease cut site is designed to allow for cleavage, via a restriction endonuclease, of a 3' portion of said infectious nucleic acid, thereby creating a restriction endonuclease-cleaved infectious nucleic acid, optionally wherein said restriction endonuclease-cleaved infectious nucleic acid encodes a coxsackievirus comprising a 3' end with less than 10 ribonucleotides that are not present in a picornavirus genome, or optionally wherein said restriction endonuclease-cleaved infectious nucleic acid encodes a coxsackievirus comprising a 3' end with no ribonucleotides that are not present in a picornavirus genome.

7. The isolated infectious nucleic acid of any one of claims 1-2, wherein said infectious nucleic acid is RNA, said infectious nucleic acid encodes a ribozyme, and said infectious nucleic acid comprises a restriction endonuclease cut site.

8. The isolated infectious nucleic acid of claim 7, wherein said ribozyme is designed to cleave a 5' portion of RNA from an RNA molecule transcribed from said infectious nucleic acid, thereby creating a ribozyme-cleaved RNA; or wherein said ribozyme-cleaved RNA comprises a 5' end with no ribonucleotides that are not present in a picornavirus genome; or wherein said restriction endonuclease cut site is designed to allow for cleavage, via a restriction endonuclease, of a 3' portion of RNA from said infectious nucleic acid, thereby creating a restriction endonuclease-cleaved infectious nucleic acid, optionally wherein said restriction endonuclease-cleaved infectious nucleic acid encodes a coxsackievirus comprising a 3' end with less than 10 ribonucleotides that are not present in a picornavirus genome, or optionally wherein said restriction endonuclease-cleaved infectious nucleic acid encodes a coxsackievirus comprising a 3' end with no ribonucleotides that are not present in a picornavirus genome.

9. A composition comprising an effective amount of infectious nucleic acid encoding a coxsackievirus, wherein said infectious nucleic acid lacks at least 20 nucleotides from position 631 to position 698 of a wild-type coxsackievirus A21 genome, and wherein said infectious nucleic acid comprises a microRNA target element for a muscle-specific microRNA that is located between a VI domain and an authentic translation start site of said infectious nucleic acid, for use in a method for treating cancer present in a mammal, the method comprising administering said composition to said mammal.

10. The composition for use of claim 9, wherein said mammal is a human.

11. The composition for use of any one of claims 9-10, wherein said cancer is melanoma, myeloma, or breast cancer.

12. The composition for use of any one of claims 9-11, wherein said infectious nucleic acid lacks said nucleotides from position 631 to position 698; and/or wherein said muscle-specific microRNA is miR-133 or miR-206; and/or wherein said infectious nucleic acid comprises said microRNA target element between a first position and a second position, wherein said first position corresponds to position 631 of said wild-type coxsackievirus A21 genome, and wherein said second position corresponds to position 699 of said wild-type coxsackievirus A21 genome.

13. The composition for use of any one of claims 9-12, said method further comprising administering an anti-cancer agent to said mammal.

14. The composition for use of claim 13, wherein said anti-cancer agent is a checkpoint inhibitor.

15. The composition for use of claim 14, wherein said checkpoint inhibitor is selected from the group consisting of a CTLA-4 inhibitor, a PD-1 inhibitor, and a PD-L1 inhibitor.
